# EUROPEAN PATENT SPECIFICATION

(11) **EP 3 760 258 B1**
(45) Date of publication and mention of the grant of the patent: **21.02.2024**
(21) Application number: 19775029.2
(22) Date of filing: 26.03.2019
(51) Int. Cl.: A61M 5/315

(54) **LIQUID MEDICINE EJECTION TOOL OR GASKET PRESSING TOOL FOR SYRINGE, AND LIQUID MEDICINE ADMINISTRATION TOOL PROVIDED THEREWITH**
AUSSTOSSINSTRUMENT FÜR FLÜSSIGE MEDIZIN ODER DICHTUNGSPRESSINSTRUMENT FÜR EINE SPRITZE UND DAMIT VERSEHENES INSTRUMENT ZUR VERABREICHUNG VON FLÜSSIGER MEDIZIN
OUTIL D'ÉJECTION DE MÉDICAMENT LIQUIDE OU OUTIL DE COMPRESSION DE JOINT D'ÉTANCHÉITÉ POUR SERINGUE, ET OUTIL D'ADMINISTRATION DE MÉDICAMENT LIQUIDE ÉQUIPÉ DE CEUX-CI

(30) Priority: 28.03.2018 JP 2018063371; 29.03.2018 JP 2018066279; 29.03.2018 JP 2018066280
(43) Date of publication of application: 06.01.2021
(73) Proprietor: TERUMO Kabushiki Kaisha, Tokyo 151-0072 (JP)
(72) Inventor: OKIHARA, Hitoshi, Fujinomiya-shi, Shizuoka 418-0004 (JP)
(74) Representative: TBK
(86) International application number: PCT/JP2019/012928
(87) International publication number: WO 2019/189215

(56) References cited:
- EP-A1- 1 166 807
- EP-A1- 1 849 490
- WO-A1-2018/052101
- WO-A1-2018/052101

## Description

The present invention relates to a gasket pressing tool for a syringe or a liquid medicine ejection tool, and a liquid medicine administration tool including the same.

### Background Art

Conventionally, a so-called threaded syringe, configured to advance a plunger by a screwing operation of a screw in order to discharge a relatively high-viscosity liquid medicine from a syringe barrel, has been proposed. Examples of such a type of injector include, for example, JP 2007-520276 A (family document: US 2007-287958 A1), JP 2008-167954 A, and JP 2005-535415 A.

In an injector 3 of JP 2007-520276 A, a threaded rod 1 is attached to a plunger 6 at its distal end. The plunger 6 is located within a syringe body 4 having a proximal flange 8. A cap 5 is connected to the flange 8. A handle 2 which acts as a thumbwheel for turning the rod and advancing the plunger 6, is attached at a proximal end of the rod 1. A stopper 7 is located within the syringe body 4 to form an air-tight seal when a force of the advancing plunger is applied against a viscous material within the syringe body. The viscous material is ejected through the distal end of the syringe body into an appropriate receiving device.

In syringes including the injector as disclosed in JP 2007-520276 A, a gasket made of an elastic material is used as the above-described stopper.

Recently, endoscopic mucosal resection using local injection of a highly viscous substance has been performed. As the highly viscous substance, hyaluronic acid, which is a medicine, is generally used. As the endoscopic mucosal resection, for example, partial resection of an inner wall of a digestive tract using an endoscope is performed.

In the endoscopic mucosal resection, it is important to control the injection amount of hyaluronic acid. However, the hyaluronic acid, which is the highly viscous substance, is hardly administered unless being extruded with a considerable pressure. In addition, there is a case where the hyaluronic acid is not immediately administered even after performing the administration operation.

The inventors of the present application have conducted earnest studies and found that a time lag between the administration operation and actual outflow of the highly viscous substance is caused by a deformation of a gasket. Since the gasket is elastically deformed, the highly viscous substance does not flow out until the elastic deformation is completed since the start of injection. It has been found that it is difficult to favorably perform the administration of the highly viscous substance to a target site due to this time lag.

The object of the present invention is to provide a liquid medicine ejection tool and a liquid medicine administration tool including the same which can reduce a time lag between an administration operation of a liquid medicine and actual outflow of the liquid medicine and administer the liquid medicine to a target site in a timely and favorable manner even when the liquid medicine is a highly viscous substance.

In addition, in a syringe of JP 2008-167954 A, a threaded flange 4 having a female screw 3 is mounted on a syringe barrel 1, a male screw 7 formed on a shaft portion 6 of a plunger 5 is screwed into a female screw 3 of the threaded flange 4, and a click protrusion 9 is engaged with an axial groove 8 formed in the shaft portion of the plunger 5 every time the shaft portion 6 of the plunger 5 is rotated by a predetermined angle. The syringe includes: a movable piece 10 which is provided on the outer peripheral side of the shaft portion 6 of the plunger 5 to be movable in a radial direction and has the click protrusion 9; and a pressing piece 11 which presses and moves the movable piece 10 toward the shaft portion 6 side of the plunger 5 so that the click protrusion 9 is engaged with the axial groove 8 formed in the shaft portion 6 of the plunger 5.

Further, the threaded flange 4 has slits 16 at both ends with which the flange 2 of the syringe barrel 1 fits. The threaded flange 4 is engaged and integrated with the flange 2 by being rotated to a position overlapping the flange 2, and is detached from the flange 2 by being rotated to a position where the threaded flange 4 intersects the flange 2 around 90°.

The female screw 3 is formed at the center of the threaded flange 4, and the male screw 7 of the shaft portion 6 of the plunger 5 is screwed into the female screw 3. In the plunger 5, a gasket 18 is mounted to a distal end of the shaft portion 6, and the male screw 7 of the shaft portion 6 is screwed into the female screw 3 of the threaded flange 4. Further, the plunger 5 is configured such that ejection is performed every time the shaft portion 6 of the plunger 5 rotates by a predetermined angle by rotating the shaft portion 6 of the plunger 5 via a handle 19 in a state where the threaded flange 4 is mounted to the flange 2 of the syringe barrel 1.

When hyaluronic acid, which is a highly viscous substance, is administered using the syringe as disclosed in JP 2008-167954 A, the plunger 5 advances by rotating the plunger 5, the gasket 18 advances, and the highly viscous substance is discharged. The gasket 18 and the plunger 5 are pushed rearward by the resistance generated at the time of pressing the highly viscous substance.

As the present inventor have conducted studies, it has been found that a pressing force to the rear side during the pressing the above-described highly viscous substance is applied to a connecting portion that accommodates the flange of the syringe barrel to deform the connecting portion to be expanded. It has been found that a time lag between an administration operation and actual outflow of the highly viscous substance is caused by a deformation of the connecting portion, specifically, the highly viscous substance does not flow out until the deformation of the connecting portion is completed since the start of injection. It has been found that it is difficult to favorably perform the administration of the highly viscous substance to a target site due to this time lag.

It would be useful to provide a gasket pressing tool for a syringe and a liquid medicine administration tool including the same which can administer a highly viscous substance to a target site in a timely and favorable manner with a short time lag between an administration operation of the highly viscous substance and actual outflow of the highly viscous substance.

In addition, a threaded syringe of JP 2005-535415 A is provided with a mechanism for generating a click sound when a plunger rotates in order to provide a clinician with a tactile or audible indication for the rotation of the plunger. In this mechanism, the click sound is generated by an impact when a click piece engages with a groove provided in the plunger.

In the mechanism of JP 2005-535415 A, the magnitude of the generated click sound differs depending on a rotation speed of the plunger, and almost no click sound is generated when the plunger is slowly rotated.

In addition, recently, endoscopic mucosal resection using local injection of a highly viscous substance has been performed. As the highly viscous substance, hyaluronic acid, which is a medicine, is generally used. As the endoscopic mucosal resection, for example, partial resection of an inner wall of a digestive tract using an endoscope is performed. In the endoscopic mucosal resection, an endoscopic operation and injection of the highly viscous substance are performed by different operators. For this reason, it is difficult for an endoscope operator to grasp the injection amount of the highly viscous substance. With a medicine administration tool that generates the click sound as described above, the injection amount can be grasped with the click sound. In this case, however, the click sound is preferably generated only during the injection, and is required not to be generated during reverse rotation or idling, for example.

It would be useful to provide a gasket pressing tool for a syringe and a liquid medicine administration tool including the same which can generate a certain volume of sound every time a plunger is rotated by a predetermined amount during a medicine ejection operation and generate the sound only when pressing the gasket to move forward.

A prior art syringe is disclosed in WO 2018/052101 A1. Prior art gaskets and gasket pressing tools are further disclosed in EP 1 849 490 A1 and EP 1 166 807 A1.

The object of the invention is achieved by a liquid medicine ejection tool according to claim 1. Advantageous embodiments are carried out according to the dependent claims.

A liquid medicine ejection tool provided with: a syringe that includes a barrel having a flange at a proximal end portion and a gasket slidable inside the barrel and made of an elastic material; and a gasket pressing tool used by being mounted on the syringe, wherein
the gasket includes a tubular portion, a distal closing portion that closes a distal end of the tubular portion, a lumen portion defined by the tubular portion and the distal closing portion, and an annular rib provided on an outer surface of the tubular portion,
the gasket pressing tool includes: a gasket pressing member having a gasket-mounting portion, which is capable of entering the lumen portion of the gasket and made of a material harder than the gasket, and a gasket proximal surface pressing portion provided at a proximal end portion of the gasket-mounting portion; a syringe-mounting member that movably accommodates the gasket pressing member and has a proximal barrel-mounting portion that is mountable to a proximal end portion of the barrel having the flange; and a plunger that has a distal end portion entering the syringe-mounting member and is capable of pressing the gasket pressing member in a distal direction,
the gasket has an original lumen portion axial length S when the gasket is not inserted into the barrel and the gasket pressing member is not mounted, the gasket-mounting portion of the gasket pressing member has an axial length T, and the axial length T is longer than the original lumen portion axial length S, and
when the gasket is inserted into the barrel and the gasket pressing member is mounted, a mounting portion distal surface of the gasket-mounting portion of the gasket pressing member abuts on a lumen distal surface of the lumen portion of the gasket, and the gasket proximal surface pressing portion of the gasket pressing member becomes close to or abuts on a proximal surface of the gasket.

In addition, a liquid medicine administration tool may include: the above-described liquid medicine ejection tool; a liquid medicine charged in the syringe; and a sealing member sealing the distal end portion of the barrel.

### Brief Description of Drawings

Fig. 1 is a front view of a liquid medicine ejection tool according to an embodiment of the present invention.
Fig. 2 is a cross-sectional view taken along line A-A of Fig. 1.
Fig. 3 is a front view of the liquid medicine ejection tool according to the embodiment of the present invention in a state where a gasket pressing assembly is mounted on a gasket.
Fig. 4 is a left side view of the liquid medicine ejection tool illustrated in Fig. 3.
Fig. 5 is an enlarged bottom view of the liquid medicine ejection tool illustrated in Fig. 3.
Fig. 6 is an enlarged cross-sectional view taken along line B-B of Fig. 3.
Fig. 7 is a cross-sectional view taken along line C-C of Fig. 3.
Fig. 8 is a cross-sectional view taken along line D-D of Fig. 4.
Fig. 9 is a perspective view of the liquid medicine ejection tool illustrated in Fig. 3.
Fig. 10 is an explanatory view for describing components used in the liquid medicine ejection tool illustrated in Fig. 3.
Fig. 11 is a front view of the gasket pressing assembly used in the liquid medicine ejection tool illustrated in Fig. 3.
Fig. 12 is a bottom view of the gasket pressing assembly illustrated in Fig. 11.
Fig. 13 is a cross-sectional view taken along line E-E of Fig. 11.
Fig. 14 is an enlarged view of the vicinity of the gasket of Fig. 7.
Fig. 15 is a front view of the gasket used in the liquid medicine ejection tool of the present invention.
Fig. 16 is a bottom view of the gasket illustrated in Fig. 15.
Fig. 17 is a vertical cross-sectional view of the gasket illustrated in Fig. 15 (when the gasket is not inserted into a barrel and the gasket pressing member is not mounted).
Fig. 18 is an explanatory view for describing a gasket shape when the gasket used in the liquid medicine ejection tool of the present invention is inserted into the barrel and the gasket pressing member is mounted.
Fig. 19 is a front view of a gasket pressing member used in the gasket pressing assembly illustrated in Fig. 11.
Fig. 20 is a cross-sectional view taken along line F-F of Fig. 19.
Fig. 21 is a bottom view of the gasket pressing member illustrated in Fig. 19.
Fig. 22 is a perspective view of the gasket pressing member illustrated in Fig. 19 as seen obliquely from below.
Fig. 23 is a front view of a sound generating member used in the gasket pressing assembly illustrated in Fig. 11.
Fig. 24 is a bottom view of the sound generating member illustrated in Fig. 23.
Fig. 25 is a cross-sectional view taken along line G-G of Fig. 23.
Fig. 26 is a view obtained by illustrating a left side view of a syringe-mounting member used in the liquid medicine ejection tool illustrated in Fig. 3 sideways.
Fig. 27 is a bottom view of the syringe-mounting member in the state illustrated in Fig. 26.
Fig. 28 is a cross-sectional view taken along line H-H of Fig. 26.
Fig. 29 is a cross-sectional view taken along line I-I of Fig. 27.
Fig. 30 is a reference view of the syringe-mounting member of Fig. 26.
Fig. 31 is an explanatory view of an end surface along line Y-Y of Fig. 30.
Fig. 32 is an explanatory view of an end surface along line X-X of Fig. 30.
Fig. 33 is an explanatory view of an end surface along line Z-Z of Fig. 30.
Fig. 34 is a perspective view of the gasket pressing member in the state illustrated in Fig. 26 as seen obliquely from above.
Fig. 35 is a perspective view of the gasket pressing member in the state illustrated in Fig. 26 as seen obliquely from below.
Fig. 36 is an enlarged front view of a plunger used in the liquid medicine ejection tool illustrated in Fig. 3.
Fig. 37 is a left side view of the plunger illustrated in Fig. 36.
Fig. 38 is an enlarged perspective view of a distal end portion of the plunger illustrated in Fig. 36.
Fig. 39 is an explanatory cross-sectional view for describing a sound generating mechanism in the liquid medicine ejection tool of the present invention.
Fig. 40 is an explanatory cross-sectional view for describing the sound generating mechanism in the liquid medicine ejection tool of the present invention.
Fig. 41 is an explanatory cross-sectional perspective view for describing the sound generating mechanism in the liquid medicine ejection tool of the present invention.
Fig. 42 is an explanatory cross-sectional view for describing the sound generating mechanism in the liquid medicine ejection tool of the present invention.
Fig. 43 is an explanatory cross-sectional view for describing the sound generating mechanism in the liquid medicine ejection tool of the present invention.
Fig. 44 is an explanatory cross-sectional perspective view for describing the sound generating mechanism of the present invention.
Fig. 45 is a front view of a liquid medicine ejection tool according to another embodiment of the present invention in a state where a gasket pressing assembly is mounted on a gasket.
Fig. 46 is a vertical cross-sectional view of the liquid medicine ejection tool illustrated in Fig. 45.
Fig. 47 is a perspective view of the liquid medicine ejection tool illustrated in Fig. 45.
Fig. 48 is a view obtained by illustrating a left side view of a syringe-mounting member used in the liquid medicine ejection tool illustrated in Fig. 45 sideways.
Fig. 49 is a bottom view of the syringe-mounting member in the state illustrated in Fig. 48.
Fig. 50 is a cross-sectional view taken along line J-J of Fig. 48.
Fig. 51 is a cross-sectional view taken along line K-K of Fig. 49.
Fig. 52 is a left side view of the syringe-mounting member in the state illustrated in Fig. 48.
Fig. 53 is a perspective view of the syringe-mounting member in the state illustrated in Fig. 48 as seen from the distal side.
Fig. 54 is a perspective view of the syringe-mounting member in the state illustrated in Fig. 48 as viewed from the proximal side.
Fig. 55 is an enlarged vertical cross-sectional view of a gasket pressing assembly used in the liquid medicine ejection tool illustrated in Fig. 45.
Fig. 56 is an explanatory view for describing components of the gasket pressing assembly used in the liquid medicine ejection tool illustrated in Fig. 45.
Fig. 57 is an enlarged bottom view of a gasket pressing member used in the liquid medicine ejection tool illustrated in Fig. 45.
Fig. 58 is an enlarged plan view of a joint member used in the liquid medicine ejection tool illustrated in Fig. 45.
Fig. 59 is an enlarged front view of a plunger used in the liquid medicine ejection tool illustrated in Fig. 45.
Fig. 60 is an enlarged view of a distal end portion of the plunger illustrated in Fig. 59.
Fig. 61 is a vertical cross-sectional view of a liquid medicine administration tool according to still another embodiment of the present invention.
Fig. 62 is an explanatory view for describing a treatment method using the liquid medicine administration tool of the present invention.
Fig. 63 is an explanatory view for describing the treatment method using the liquid medicine administration tool of the present invention.
Fig. 64 is an explanatory view for describing the treatment method using the liquid medicine administration tool of the present invention.
Fig. 65 is an explanatory view for describing the treatment method using the liquid medicine administration tool of the present invention.
Fig. 66 is an explanatory view for describing the treatment method using the liquid medicine administration tool of the present invention.

### Description of Embodiments

A gasket pressing tool for a syringe, a liquid medicine ejection tool, and a liquid medicine administration tool of the present invention will be described with reference to embodiments illustrated in the drawings.

As a first configuration of the present invention, a liquid medicine ejection tool 1 includes: a syringe 2 that includes a barrel 20 having a flange 23 at its proximal end portion and a gasket 7 slidable in the barrel 20 and elastically deformable; and a gasket pressing tool (gasket pressing tool for a syringe) 3 used by being mounted on the syringe 2.

The gasket 7 includes a tubular portion 72, a distal closing portion 71 closing a distal end of the tubular portion 72, a lumen portion 70 defined by the tubular portion 72 and the distal closing portion 71, and an annular rib provided on an outer surface of the tubular portion 72.

The gasket pressing tool 3 includes: a gasket pressing member 8 having a gasket-mounting portion (gasket entering portion) 80, which can enter the lumen portion 70 of the gasket 7 and made of a material harder than the gasket 7, and a gasket proximal surface pressing portion (flange portion) 81 provided at its proximal end portion of the gasket-mounting portion 80; a syringe-mounting member 4 that movably accommodates the gasket pressing member 8 and has a proximal barrel-mounting portion (barrel mounting slit) 43 which can be mounted to a proximal end portion of the barrel 20 having the flange 23; and a plunger 5 that has a distal end portion entering the syringe-mounting member 4 and can press the gasket pressing member 8 in a distal direction.

The gasket 7 has an original lumen portion axial length S when the gasket 7 is not inserted into the barrel 20 and the gasket pressing member 8 is not mounted, the gasket-mounting portion 80 of the gasket pressing member 8 has an axial length T, and the axial length T is longer than the original lumen portion axial length S.

When the gasket 7 is inserted into the barrel 20 and the gasket pressing member 8 is mounted, a mounting portion distal surface 80a of the gasket-mounting portion 80 of the gasket pressing member 8 abuts on a lumen distal surface 76 of the lumen portion 70 of the gasket 7, and the gasket proximal surface pressing portion 81 of the gasket pressing member 8 becomes close to or abuts on a proximal surface of the gasket 7.

In addition, the liquid medicine administration tool of the present invention includes: the liquid medicine ejection tool 1 described above; a liquid medicine 11 charged in the syringe 2; and a sealing member 21 sealing the distal end portion of the barrel 20.

In addition, in the liquid medicine ejection tool 1 of the present embodiment, when the gasket 7 is inserted into the barrel 20 and the gasket pressing member 8 is mounted, an inner surface 77 of the lumen portion 70 of the gasket 7 is in close contact with an outer surface of the gasket-mounting portion 80 of the gasket pressing member 8, the gasket 7 is turned into a state of being stretched in the axial direction by the gasket pressing member 8, and the gasket proximal surface pressing portion 81 of the gasket pressing member 8 is turned into a state of being capable of pressing a proximal surface 79 of the gasket 7.

As a second configuration of the present invention, a gasket pressing tool 3 for a syringe is used by being mounted to a syringe 2 that includes: a barrel 20 having an annular flange 23 at its proximal end portion; and a gasket 7 slidably accommodated in the barrel 20.

The gasket pressing tool 3 for a syringe includes: a gasket pressing member 8 configured to press the gasket 7; a tubular syringe-mounting member 4 that movably accommodates the gasket pressing member 8 and can be mounted on the proximal end portion of the barrel 20; and a plunger 5 that has a distal end portion entering the syringe-mounting member 4 and can press a gasket pressing assembly (gasket-mounting member) 6 having the gasket pressing member 8 by rotation.

The plunger 5 includes: a distal end portion that can press the gasket pressing member 8; a shaft portion 51 that extends from its distal end portion in a proximal direction and has a shaft-side screwing portion 55 on its outer peripheral surface; and a handle 52 configured to rotate the plunger provided at the proximal end portion of the shaft portion 51.

The syringe-mounting member 4 includes: a body portion (barrel-mounting portion) 40 that can accommodate the flange 23 of the barrel 20; a tubular accommodation portion 42 that extends from a proximal end of the body portion 40 in the proximal direction of the gasket pressing tool 3 and can accommodate the gasket pressing member; and a tubular insertion portion 110 (lumen 45) which extends from a proximal end of the tubular accommodation portion 42 (accommodation portion 47) in the proximal direction of the gasket pressing tool 3 and into which the shaft portion 51 of the plunger 5 is inserted. The insertion portion 110 (lumen 45) of the syringe-mounting member 4 has an insertion-portion-side screwing portion 46, which is screwed with the shaft-side screwing portion of the shaft portion 51, on its inner peripheral surface.

The body portion 40 includes: a distal-side support portion 103 that can support a distal surface of the flange 23 (in other words, can abut on the distal surface of the flange 23); a proximal-side support portion 104 that can support a proximal surface of the flange 23 (in other words, can abut on the proximal surface of the flange 23); a connecting portion 105 that connects the distal-side support portion 103 and the proximal-side support portion 104; a flange accommodation portion 102 that is formed of the distal-side support portion 103, the proximal-side support portion 104, and the connecting portion 105 and can accommodate the flange 23; and a flange insertion opening 101 that is open to a lateral side of the body portion 40 and configured to insert the flange into the flange accommodation portion 102.

The distal-side support portion 103 has an axial thickness (N) of 5 to 25 mm along a central axis of the syringe-mounting member 4, and the proximal-side support portion 104 has an axial thickness (M) of 5 to 25 mm along the central axis of the syringe-mounting member 4. In addition, the connecting portion 105 has a radial width (Ta) of 1 to 5 mm along the radial direction of the syringe-mounting member 4.

In addition, a liquid medicine administration tool of the present invention may include: a so-called prefilled syringe which includes a barrel 20, a liquid medicine 11 charged in the barrel 20, a gasket 7 slidably accommodated in the barrel 20, and a sealing member (sealing cap) 21 sealing a distal end of the barrel 20; and a gasket pressing tool 3 on which the prefilled syringe is mounted.

The prefilled syringe does not necessarily include a so-called plunger. The barrel 20 includes a nozzle 22 for discharging a liquid medicine and a collar portion 24 provided so as to cover the nozzle 22 at its distal end, and includes the flange 23 protruding outward at a rear end portion.

As a third configuration of the present invention, a gasket pressing tool 3 for a syringe is used by being mounted to a syringe 2 that includes: a barrel 20; and a gasket 7 slidably accommodated in the barrel 20.

The gasket pressing tool 3 for a syringe includes: a gasket pressing assembly 6 including a gasket pressing member 8 and a sound generating member 9; a syringe-mounting member 4 which includes a proximal barrel-mounting portion 43 and movably accommodates the gasket pressing assembly 6; and a plunger 5 which enters the syringe-mounting member 4 on a distal side and can press (can press and advance) the gasket pressing assembly 6 by rotation (advancing along with rotation).

The sound generating member 9 includes claw portions 92 and 93 that are elastically deformable (specifically, elastically deformable in the circumferential direction with respect to an axis of the gasket pressing member 8). The gasket pressing member 8 includes wall portions 88 and 89 that abut on (specifically, abut on and press) the claw portions 92 and 93, respectively. The claw portions 92 and 93 include claw tip portions 92a and 93a protruding rearward from the wall portions 88 and 89, respectively. The plunger 5 includes claw-flicking ribs 54 provided on a side surface of the distal end portion. In a state where the plunger 5 (specifically, the distal end portion) does not abut on the gasket pressing assembly 6 in a pressable manner (non-pressing state), the claw-flicking rib 54 is located on the rear side of each of the claw tip portions 92a and 93a. In a state where the plunger 5 (specifically, the distal end portion) abuts on the gasket pressing assembly 6 in a pressable state (pressable state), the claw-flicking rib 54 is located on the lateral side of each of the claw tip portions 92a and 93a. As the plunger 5 is rotated, the plunger 5 advances to move the gasket pressing assembly 6 forward, the claw-flicking ribs 54 abut on the claw tip portions 92a and 93a and pass the claw portions 92 and 93 while deforming the claw portions 92 and 93. As the claw portions 92 and 93 restored after the passage abut on the wall portions 88 and 89, a sound is generated.

In addition, a liquid medicine administration tool of the present invention may include: a so-called prefilled syringe which includes a barrel 20, a liquid medicine 11 charged in the barrel 20, a gasket 7 slidably accommodated in the barrel 20, and a sealing member (sealing cap) 21 sealing a distal end of the barrel 20; and a gasket pressing tool 3 on which the prefilled syringe is mounted.

The prefilled syringe does not necessarily include a so-called plunger. The barrel 20 includes a nozzle 22 for discharging a liquid medicine and a collar portion 24 provided so as to cover the nozzle 22 at its distal end, and includes the flange 23 protruding outward at a rear end portion.

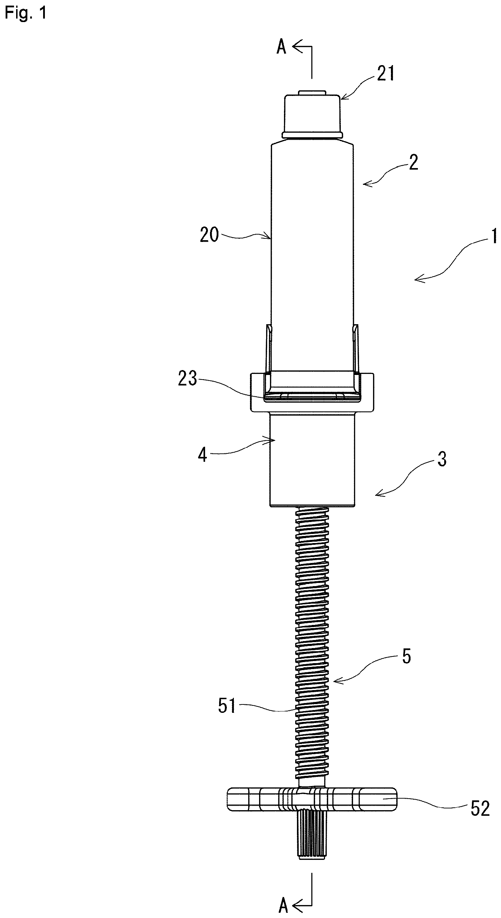

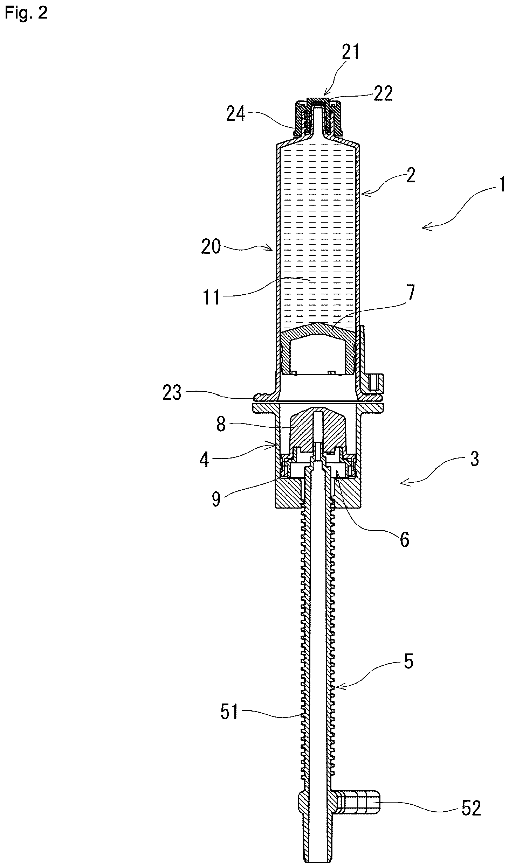

The liquid medicine administration tool (liquid medicine ejection tool 1) illustrated in Figs. 1 and 2 includes: the prefilled syringe that includes the syringe 2, the liquid medicine 11 charged in the syringe 2, and the sealing member (sealing cap) 21 sealing the distal end portion of the barrel 20; and the gasket pressing tool 3 on which the prefilled syringe is mounted. The prefilled syringe does not include a plunger. The barrel 20 includes a nozzle 22 for discharging a liquid medicine and a collar portion 24 provided so as to cover the nozzle 22 at its distal end, and includes the flange 23 protruding outward at a proximal end portion.

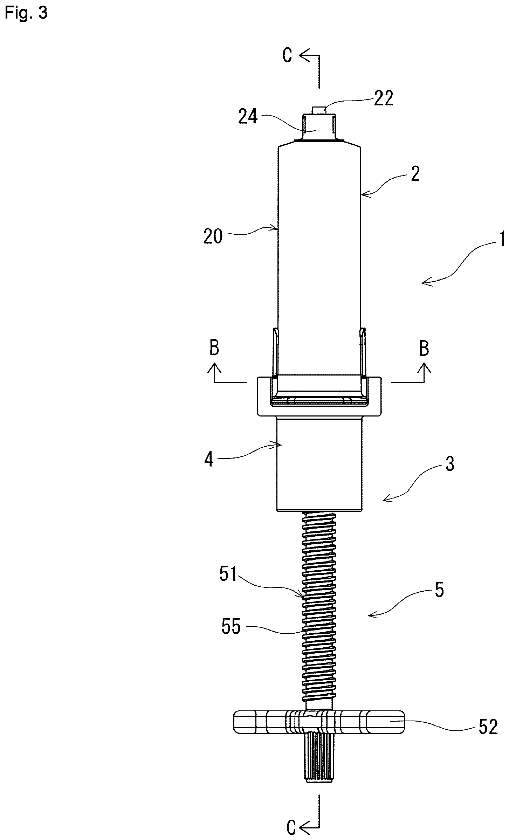

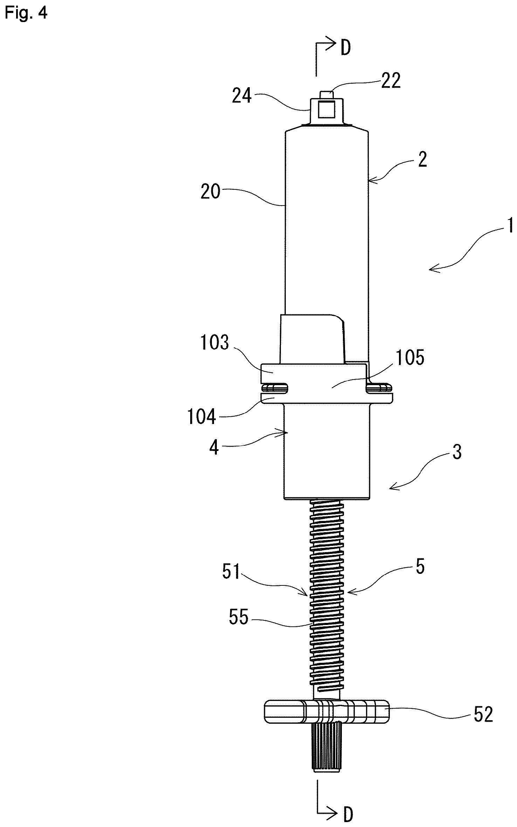

In the liquid medicine ejection tool 1 illustrated in Figs. 1 and 2, the gasket pressing assembly 6 including the gasket pressing member 8 is located on the rear side of the gasket 7 and is not mounted on the gasket 7. As the plunger 5 is rotated to move forward during use, the gasket pressing assembly 6 is mounted to the gasket 7 as illustrated in Figs. 3 and 4 to 6. The gasket pressing assembly 6 can also be referred to as a gasket-mounting member. Note that the liquid medicine and the sealing cap are not illustrated in the syringe 2 in Fig. 3 and the subsequent drawings.

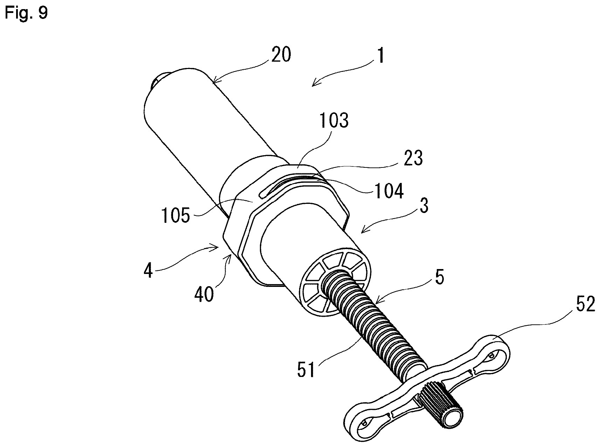

As illustrated in Figs. 1 to 9, the liquid medicine ejection tool 1 includes the gasket pressing tool 3. As described above, the gasket pressing assembly 6 is mounted to the gasket 7 in the structure illustrated in Figs. 3 and 4 to 6.

The gasket pressing tool 3 includes: the gasket pressing assembly 6; the plunger 5 configured to press the gasket pressing assembly 6 from the rear side; and the syringe-mounting member 4 configured to mount the gasket pressing assembly 6 and the plunger 5 onto the syringe 2.

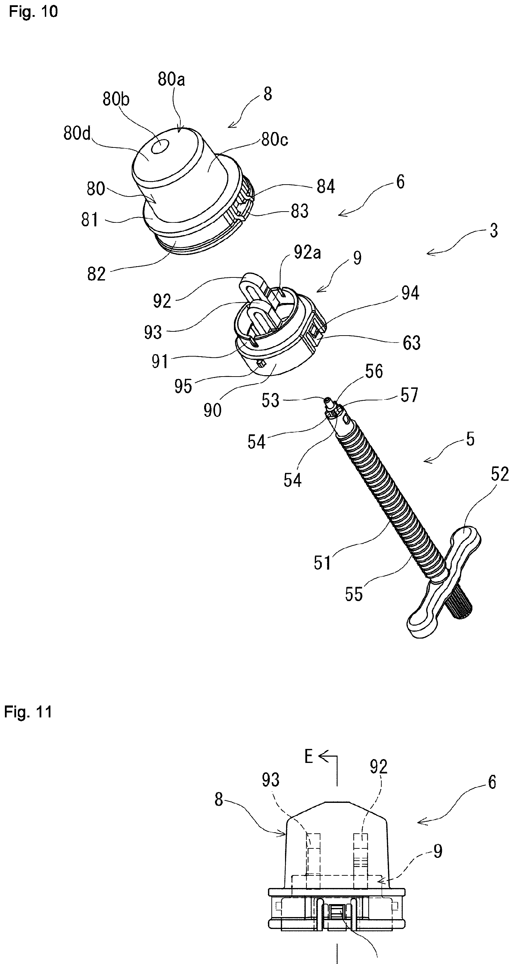

As illustrated in Figs. 10 to 13, the gasket pressing assembly 6 includes: the gasket pressing member 8 and the sound generating member 9 mounted on the gasket pressing member 8.

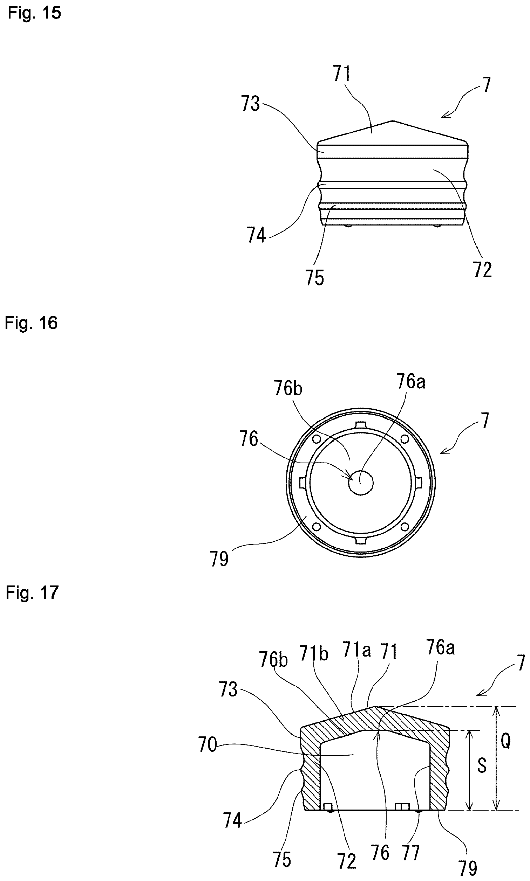

As illustrated in Figs. 15 to 18, the gasket 7 includes: the lumen portion 70; the tubular portion 72; the distal closing portion 71 that closes the distal end of the tubular portion 72; and annular ribs 73, 74, and 75 provided on an outer surface of the tubular portion 72.

The gasket 7 in Figs. 15 to 17 has a form in the state of not being inserted into the barrel 20 and having no gasket-mounting member 8 mounted thereon. As illustrated in Fig. 17, the gasket 7 has the original lumen portion axial length S when the gasket is not inserted into the syringe and the gasket-mounting member is not mounted. In addition, the lumen portion 70 of the tubular portion 72 extends in the distal direction with the substantially same inner diameter. In addition, the inner surface of the lumen portion 70 includes: the lumen distal surface 76; and a proximal-side cylindrical surface (inner surface) 77 extending rearward from a proximal end of the lumen distal surface 76 with substantially the same diameter. Further, the lumen distal surface 76 has: a flat central flat surface 76a provided at the center of the lumen distal surface; and a lumen portion tapered surface 76b extending rearward from a proximal end of the central flat surface 76a.

Specifically, as illustrated in Fig. 17, the gasket 7 has: the lumen distal surface 76 of the lumen portion 70 of the gasket 7; the flat central flat surface 76a provided at the center of the lumen distal surface; and the central flat surface 76a has a lumen portion tapered surface 76b extending rearward and radially outward from an outer edge of the central flat surface 76a. In addition, the distal closing portion 71 of the gasket 7 has: the tapered gasket distal surface 71a whose diameter is reduced toward a distal end; and a uniform-thickness portion 71b which is formed between the gasket distal surface and the lumen portion tapered surface and has a substantially uniform thickness.

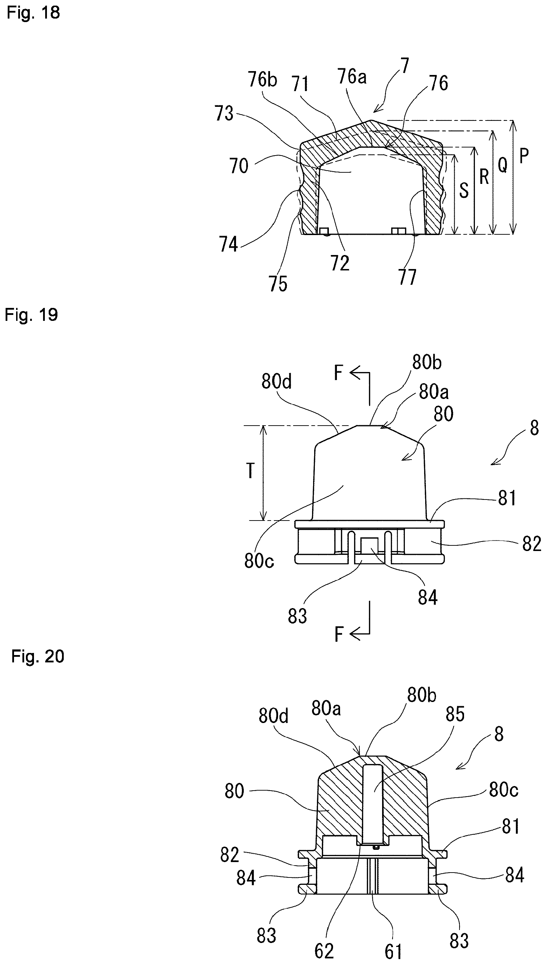

As illustrated in Figs. 19 to 22, the gasket pressing member 8 includes: the gasket-mounting portion 80 that enters the gasket 7 and comes into contact with an inner surface of the gasket; and the gasket proximal surface pressing portion 81 formed at a rear portion of the gasket-mounting portion 80. The gasket-mounting portion 80 of the gasket pressing member 8 has the axial length T, and the axial length T is longer than the original lumen portion axial length S of the gasket 7. Further, the axial length T is preferably 1.05 to 1.20 times, and particularly preferably 1.08 to 1.15 times of the original lumen portion axial length S.

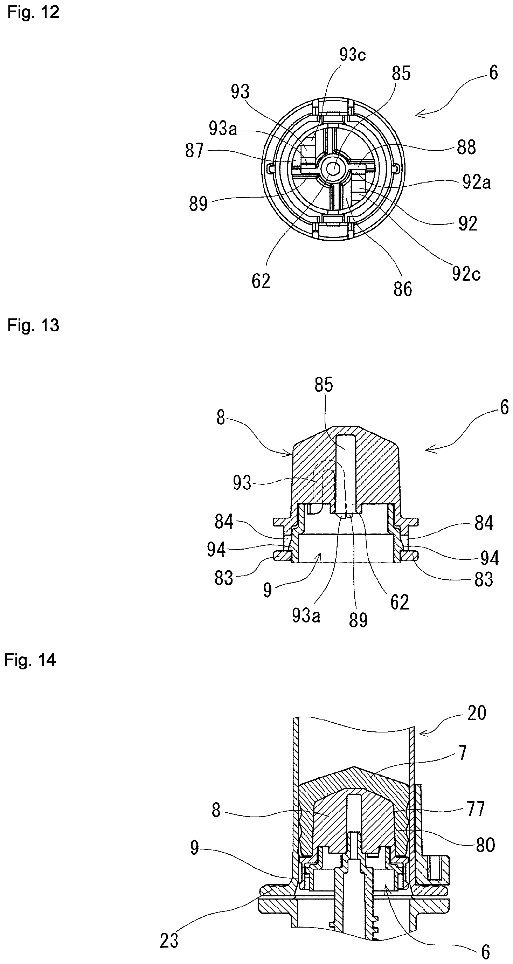

Further, as illustrated in Fig. 14, when the gasket 7 is inserted into the barrel 20 and the gasket pressing member 8 is mounted, the inner surface 77 of the lumen portion 70 of the gasket 7 is in close contact with the outer surface of the gasket-mounting portion 80 of the gasket pressing member 8, and the gasket 7 is turned into a state of being stretched in the axial direction by the gasket pressing member 8.

In addition, the gasket-mounting portion 80 includes: the distal end portion having the mounting portion distal surface 80a; and a proximal-side tapered portion 80c which extends from the distal end portion of the gasket-mounting portion 80 (the mounting portion distal surface 80a) to the gasket proximal surface pressing portion 81 and is enlarged in diameter toward the rear side to have a tapered shape. The mounting portion distal surface 80a includes a flat distal surface 80b and a distal-side tapered surface 80d extending rearward from a proximal end of the flat distal surface 80b.

When the gasket 7 is inserted into the barrel 20 and the gasket pressing member 8 is mounted, the mounting portion distal surface 80a of the gasket-mounting portion 80 of the gasket pressing member 8 abuts on the lumen distal surface 76 of the lumen portion 70 of the gasket 7.

In addition, the proximal-side tapered portion 80c of the gasket-mounting portion 80 becomes close to or abuts on an inner side surface of the lumen portion 70 of the gasket 7. In the present embodiment, the proximal-side tapered portion 80c abuts on the proximal-side cylindrical surface (inner surface) 77 of the lumen portion 70 of the gasket 7. A taper angle of the proximal-side tapered portion of the gasket-mounting portion 80 is preferably 1 to 8 degrees, and particularly preferably 3 to 6 degrees.

As illustrated in Fig. 14, when being mounted on the gasket 7, the gasket pressing member 8 is pushed into the gasket 7 to deform and stretch the gasket 7 in a state where the central flat surface 76a of the lumen distal surface 76 of the lumen portion 70 of the gasket 7 abuts on the flat distal surface 80b of the mounting portion flat surface 80a of the gasket-mounting portion 80 and the lumen portion tapered surface 76b of the lumen portion 70 abuts on the distal-side tapered surface 80d of the gasket-mounting portion 80. In addition, in the present embodiment, the entire outer surface of the gasket-mounting portion 80 has a shape that substantially coincides with the entire inner surface of the lumen portion 70 of the gasket 7 when being inserted into the barrel 20.

Specifically, the mounting portion distal surface 80a of the gasket-mounting portion 80 has: the flat distal surface 80b, which is flat, at the center of the mounting portion distal surface; and the tapered outer edge surface (distal-side tapered surface) 80d extending rearward and radially outward from the outer edge of the flat distal surface 80b. When the gasket 7 is inserted into the barrel 20 and the gasket pressing member 8 is mounted, the flat distal surface 80b of the gasket-mounting portion 80 abuts on the central flat surface 76a of the lumen portion 70 of the gasket 7, and the tapered outer edge surface 80d of the gasket-mounting portion 80 becomes close to or abuts on the lumen portion tapered surface 76b of the lumen portion 70 of the gasket.

Due to the deformation including stretching of the gasket 7, a front surface of the inner surface 77 of the lumen portion 70 of the gasket 7 is in close contact with the outer surface of the gasket-mounting portion 80 of the gasket pressing member 8. In this state, the lumen portion of the gasket 7 is not deformable practically. Even if the gasket 7 in the state where the lumen portion is not deformable is elastically deformed, the volume does not change. Accordingly, even if a needle tip for administration is removed after the end of administration, the volume of the gasket does not change, so that the outflow of the liquid medicine caused by the shape change of the gasket does not occurs.

As illustrated in Fig. 17, the gasket 7 has an original axial length Q and the original lumen portion axial length S in the state of not being inserted into the barrel 20 (syringe 2) and having no gasket-mounting member 8 mounted thereon. In addition, as illustrated in Fig. 18, the gasket 7 has a deformed axial length P and a deformed lumen portion axial length R in the state of being inserted into the syringe and having the gasket-mounting member mounted thereon. As illustrated in Fig. 18, the deformed axial length P is longer than the original axial length Q, and the deformed lumen portion axial length R is longer than the original lumen portion axial length S. The deformed axial length P is preferably longer than the original axial length Q by 0.5 to 3 mm, and particularly preferably by 1 to 2 mm. In addition, the deformed lumen portion axial length R is preferably longer than the original lumen portion axial length S by 0.5 to 3 mm, and particularly preferably by 1 to 2 mm.

As illustrated in Fig. 19, the gasket pressing member 8 has the axial length T of the gasket-mounting portion. The axial length T of the gasket pressing member 8 is preferably 1.05 to 1.20 times, and particularly preferably 1.08 to 1.15 times of the original lumen portion axial length S of the gasket 7. In addition, the taper angle of the proximal-side tapered portion 80c of the gasket-mounting portion 80 of the gasket pressing member 8 is preferably 1 to 8 degrees, and particularly preferably 3 to 6 degrees.

In addition, the gasket pressing member 8 includes a cylindrical portion 82 extending rearward from the gasket proximal surface pressing portion 81. Further, the gasket-mounting portion 80 includes a distal closing portion and a tapered diameter-reducing portion extending from the gasket proximal surface pressing portion 81 to a proximal end of the distal closing portion. The gasket pressing member 8 includes two deformable portions 83 provided in the cylindrical portion 82 and facing each other, and engagement window portions 84 provided in the respective deformable portions. The deformable portion 83 and the engagement window portion 84 form an engagement portion with respect to the sound generating member 9.

Further, the gasket pressing member 8 includes: a sound generating member accommodation portion, the axially extending wall portions 88 and 89, a plunger abutment portion 62, and a columnar recess 85 therein as illustrated in Figs. 10 to 14 and 19 to 22. The plunger abutment portion 62 of the gasket pressing member 8 can abut on and be separated from the distal end portion of the plunger 5.

Further, in the state where the gasket 7 is inserted into the barrel 20 and the gasket pressing member 8 is mounted, when the plunger 5 is advanced in the distal direction, the distal end portion of the plunger 5 abuts on the plunger abutment portion 62 of the gasket pressing member 8. When the plunger 5 is pulled in the proximal direction, the mounting state of the gasket pressing member 8 on the gasket 7 is maintained, and the plunger abutment portion 62 is separated from the distal end portion of the plunger. For this reason, the operation of the plunger 5 does not cause the gasket 7 and the gasket pressing member 8 to retract.

In addition, in the state where the gasket 7 is inserted into the barrel 20 and the gasket-mounting portion 80 is inserted into the lumen portion 70 of the gasket 7, the mounting portion distal surface of the gasket-mounting portion 80 is kept in the state of abutting on the lumen distal surface of the lumen portion 70 of the gasket 7 even if the plunger 5 is pulled in the proximal direction. For this reason, even if the pressure on the gasket-mounting portion 80 is released, the liquid medicine (highly viscous material), which has flown out once, is prevented from being drawn back into the barrel 20.

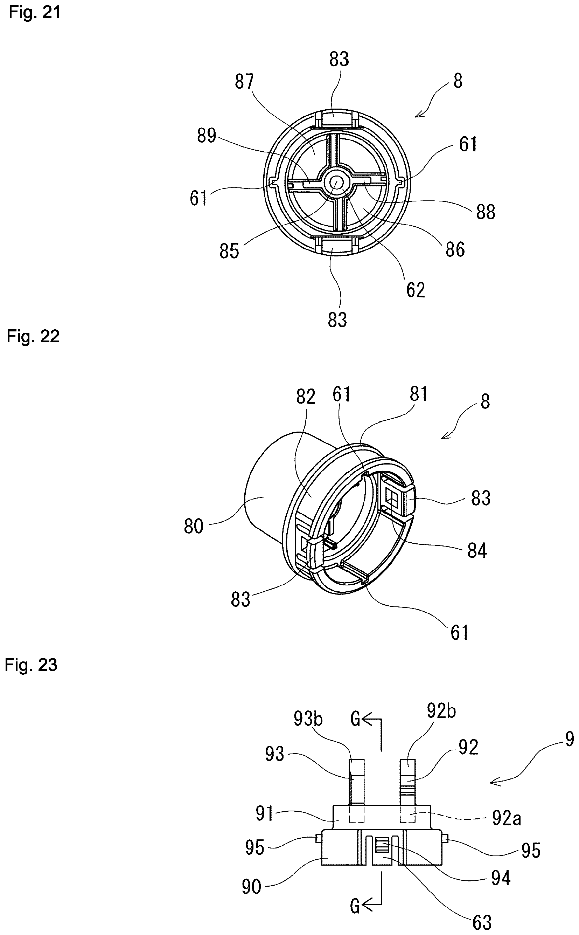

As illustrated in Fig. 21, the gasket pressing member 8 is provided with the cylindrical portion having the columnar recess 85 at the center of the inside, and provided with four partition portions extending laterally from the cylindrical portion. The four partition portions form four axially extending spaces inside the gasket pressing member 8. The wall portions 88 and 89 are formed by two partition portions facing each other with a slight shift from a central axis of the cylindrical portion formed inside the gasket pressing member. In addition, the plunger abutment portion 62 is formed by a proximal surface of the cylindrical portion having the columnar recess 85. The columnar recess 85 is a concave portion having a cylindrical shape.

The sound generating member 9 includes: a tubular body portion 91, the elastically deformable claw portions 92 and 93 protruding in the distal direction from an inner surface of the tubular body portion, and an enlarged-diameter tubular portion 90 extending downward from the tubular body portion 91 as illustrated in Figs. 10 to 13 and 23 to 25. In the present embodiment, the two claw portions 92 and 93 are provided, and the claw tip portions 92a and 93a substantially face each other.

The claw portions 92 and 93 include: base portions 92c and 93c connected to an inner surface of a distal end portion of the tubular body portion 91; and curved portions 92b and 93b protruding forward from the base portion 93c; and the claw tip portions 92a and 93a which are free ends extending from the curved portions 92b and 93b and facing the proximal direction (rear end direction). The curved portions 92b and 93b are continuous with the base portions 92c and 93c on one end side and are continuous with the claw tip portions 92a and 93a on the other end side. Further, distal end portions of the claw portions 92 and 93 are configured by the curved portions 92b and 93b. The claw tip portions 92a and 93a and the base portions 92c and 93c substantially face each other.

Further, the claw tip portions 92a and 93a are located inside the tubular body portion 91 in the present embodiment. The claw portions 92 and 93 can be elastically deformed, specifically, curved in a direction in which the claw tip portions 92a and 93a approach the base portions 92c and 93c. In addition, ends of the claw tip portions 92a and 93a are located closer to the proximal side than the base portions 92c and 93c.

In addition, the enlarged-diameter cylindrical portion 90 is provided with two deformable portions 63 facing each other and engagement ribs 94 provided on the respective deformable portions 63. The sound generating member 9 is mounted on the gasket pressing member 8 as the engagement ribs 94 of the sound generating member 9 engage with the engagement window portions 84 of the gasket pressing member 8. In addition, the enlarged-diameter cylindrical portion 90 is provided with side ribs 95, and the side ribs 95 enter two recesses 61 which are provided on the inner surface of the cylindrical portion 82 of the gasket pressing member 8 and face each other. For this reason, the rotation of the sound generating member 9 inside the gasket pressing member 8 is regulated.

Then, the distal end portion of the claw tip portion 93a of the sound generating member 9 is located on the rear side (in other words, protrudes) from a lower end of the wall portion 89 of the gasket pressing member 8 in the gasket pressing assembly 6 as illustrated in Fig. 13. Similarly, the distal end portion of the claw tip portion 92a of the sound generating member 9 is also located on the rear side (in other words, protrudes) from a lower end of the wall portion 88 of the gasket pressing member 8.

Further, tip-side side portions of the claw portions 92 and 93 of the sound generating member 9 abut on the wall portions 88 and 89 of the gasket pressing member 8, and are pressed by the wall portions 88 and 89 to be slightly curved (elastically deformed) toward the base portions 92c and 93c as illustrated in Fig. 12. In addition, the claw portions 92 and 93 are accommodated in space portions 86 and 87 divided by the partition portions inside the gasket pressing member 8.

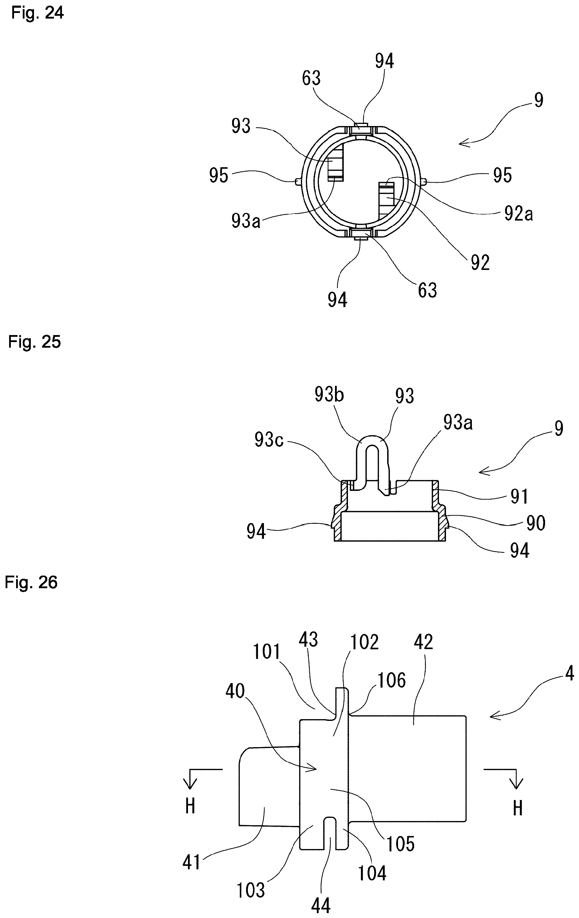

In addition, the claw tip portions 92a and 93a are tapered in the present embodiment as illustrated in Figs. 25, 40 and 41. In addition, abutment surfaces of the wall portions 88 and 89 and the claw portions 92 and 93 are formed as flat surfaces to generate a favorable sound. Specifically, surfaces of the claw tip portions 92a and 93a that respectively face the wall portions 88 and 89 (the surfaces that abut on the claw-flicking ribs 54 during rotation of the plunger 5 in a screw advancing direction to be described later) are flat surfaces. In addition, the flat surfaces of the claw tip portions 92a and 93a that abut on the claw-flicking ribs 54 are substantially parallel to a central axis of the sound generating member 9.

In addition, surfaces of the claw tip portions 92a and 93a close to the base portions 92c and 93c (the surfaces with which the claw-flicking ribs 54 are likely to come into contact during rotation of the plunger 6 in a screw retracting direction of to be described later) are formed as inclined surfaces that move away from the base portions 92c and 93c toward distal ends. In addition, the claw portions 92 and 93 extend upward from the base portions 92c and 93c and are curved in a U shape at the curved portions 92b and 93b, and the claw tip portions 93a and 93a are the free ends facing the proximal direction as illustrated in Figs. 10 and 25. Further, portions, each close to the curved portion by a predetermined length, of the claw tip portions 92a and 93a are formed to be slightly thicker than the other portions. Each of the claw portions 92 and 93 has a rectangular cross section as a whole.

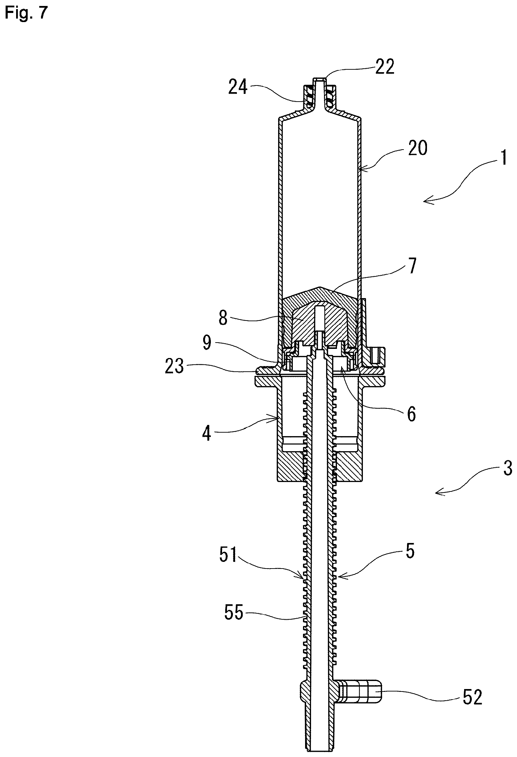

The syringe-mounting member 4 includes the barrel-mounting portion 43 as illustrated in Figs. 1 to 9 and 26 to 35, and movably accommodates the gasket pressing assembly 6, particularly as illustrated in Figs. 2 and 7.

The syringe-mounting member 4 includes the distal-side support portion 103, the proximal-side support portion 104, the connecting portion 105, the flange accommodation portion 102, and the flange insertion opening 101 as illustrated in Figs. 26 to 29, 34, and 35. The distal-side support portion 103 can abut on a distal surface of the flange 23 of the barrel 20 accommodated in the flange accommodation portion 101, and supports the distal surface of the flange 23.

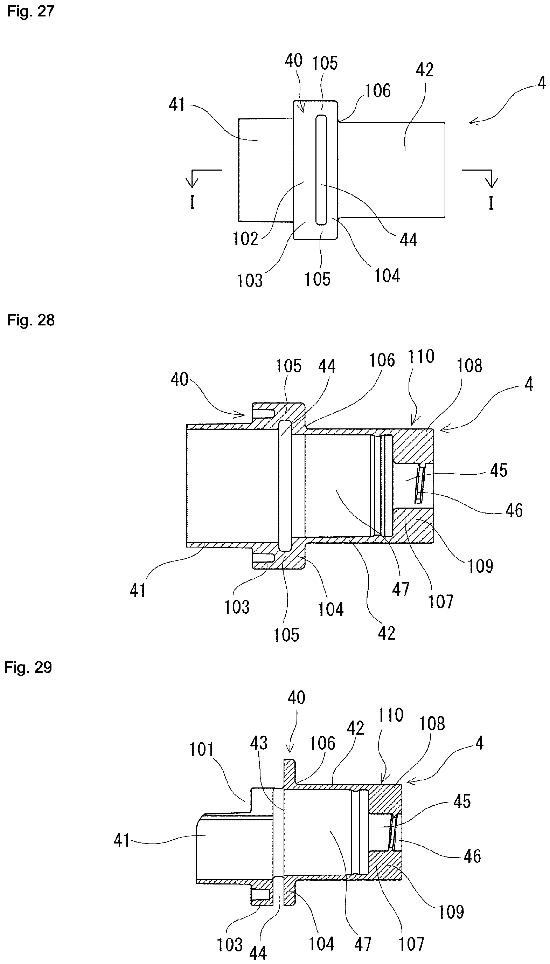

In the syringe-mounting member 4 of the present embodiment, the distal-side support portion 103 has a predetermined thickness (axial thickness: N) extending along the central axis of the syringe-mounting member 4 as illustrated in Figs. 28 to 30, and the thickness (N) is preferably 5 to 25 mm, and particularly preferably 10 to 15 mm.

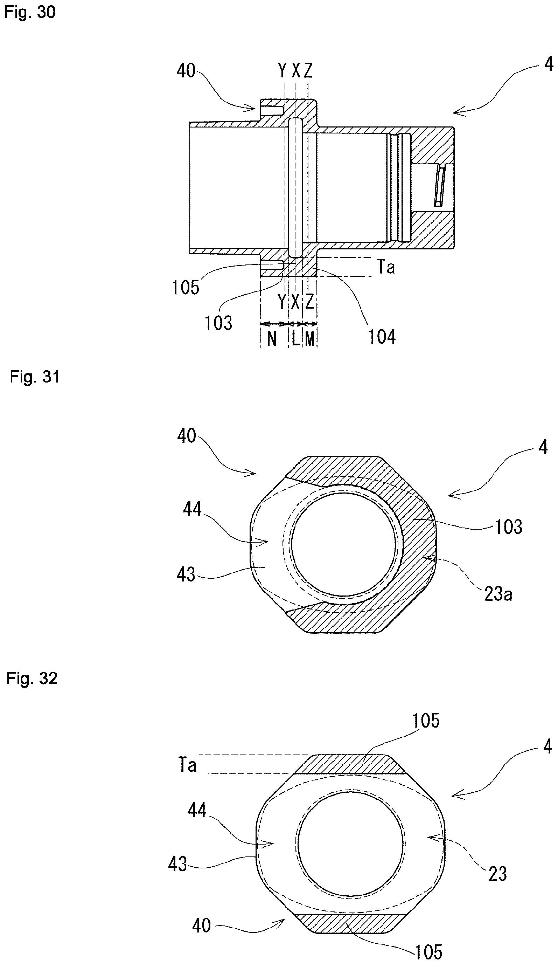

In addition, the distal-side support portion 103 has a shape and an area as illustrated in Fig. 31, which is a reference view illustrating an end surface along line Y-Y of Fig. 30, in the present embodiment. For this reason, the distal-side support portion 103 can abut on the flange 23 with a certain area as illustrated in Fig. 31. For this reason, the distal-side support portion 103 can sufficiently support the distal surface of the flange. The distal-side support portion 103 is preferably capable of supporting 55 to 80% of a distal surface 23a of the flange 23 (in other words, capable of abutting on 55 to 80% of the distal surface 23a), and particularly preferably capable of supporting 70 to 80% (capable of abutting on 70 to 80%) of the distal surface 23a.

In addition, in the syringe-mounting member 4 of the present embodiment, the proximal-side support portion 104 has a predetermined thickness (axial thickness: M) extending along the central axis of the syringe-mounting member 4 as illustrated in Figs. 28 to 30, and the thickness (M) is preferably 5 to 25 mm, and particularly preferably 10 to 15 mm.

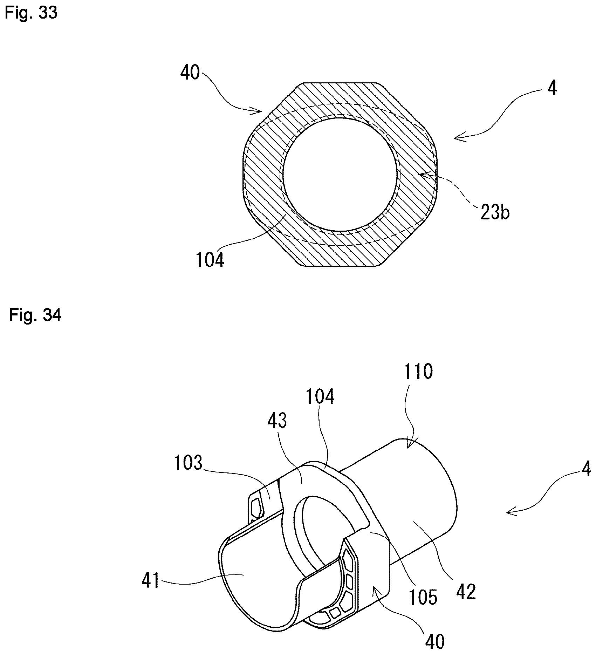

In addition, the proximal-side support portion 104 has a shape and an area as illustrated in Fig. 33, which is a reference view illustrating an end surface along line Z-Z of Fig. 30, in the present embodiment. For this reason, the proximal-side support portion 104 can abut on almost the entire proximal surface 23b of the flange 23 as illustrated in Fig. 33. For this reason, the proximal-side support portion 104 can sufficiently support the proximal surface of the flange.

In addition, the distal-side support portion 103 and the proximal-side support portion 104 are connected by the connecting portion 105 in the syringe-mounting member 4 of the present embodiment. Further, the connecting portion 105 has a predetermined thickness (axial thickness: L) extending along the central axis of the syringe-mounting member 4 as illustrated in Figs. 28 to 30, and the thickness (L) is preferably -1 to 1 mm with respect to the thickness of the flange 23 of the barrel 20, and particularly preferably -0.5 to 0.5 mm with respect to the thickness of the flange 23 of the barrel 20.

In addition, the connecting portion 105 has a shape and an area as illustrated in Fig. 32, which is a reference view illustrating an end surface along line X-X of Fig. 30, in the present embodiment, and there are two connecting portions 105 facing each other. In addition, the connecting portion 105 has the radial width Ta along the radial direction of the syringe-mounting member 4 as illustrated in Figs. 30 and 32. The radial width (Ta) is preferably 1 to 5 mm, and particularly preferably 2 to 3 mm.

The syringe-mounting member 4 of the present embodiment includes the flange accommodation portion 102 formed (defined) by the distal-side support portion 103, the proximal-side support portion 104, and the connecting portion 105. Further, the flange accommodation portion 102 includes: the flange insertion opening 101 open to the lateral side of the body portion 40 and configured to insert the flange into the flange accommodation portion 102; and a slit opening (opening portion) 44 which faces the flange insertion opening 101 (open to the lateral side of the body portion 40) and into which a part of the flange accommodated in the flange accommodation portion can enter.

The syringe-mounting member 4 includes a proximal barrel-mounting portion 43 at a proximal end of the body portion 40. The proximal barrel-mounting portion 43 accommodates the flange 23 of the barrel 20 of the syringe 2. In the present embodiment, the proximal barrel-mounting portion 43 is formed using a slit (barrel mounting slit) into which the flange 23 of the syringe 2 can be inserted from one long diameter side (in other words, vertically inserted) as illustrated in Fig. 34. For this reason, in the state where the syringe 2 is mounted on the syringe-mounting member 4, one long-diameter portion of the flange 23 and two short-diameter portions facing each other of the syringe 2 are accommodated in the syringe-mounting member 4, and a long-diameter portion on the other end side is exposed. In addition, one end of the long-diameter portion of the flange accommodated in the syringe-mounting member 4 is exposed through the slit opening 44. In the present embodiment, the proximal barrel-mounting portion 43 is formed by a vertically elongated U-shaped slit.

In addition, in the syringe-mounting member 4 of the present embodiment, the body portion 40 has an outer peripheral surface (outer wall portion) extending along the central axis of the syringe-mounting member from a distal end of the distal-side support portion 103 to a proximal end of the proximal-side support portion 104 as illustrated in Figs. 26 to 29, 34, and 35. Specifically, the outer peripheral surface (outer wall portion) has a semi-polygonal tubular shape in which a portion of the flange insertion opening 101 disappears.

In addition, in the present embodiment, the syringe-mounting member 4 includes a substantially semi-tubular portion 41 extending to the distal side from the body portion 40 having the proximal barrel-mounting portion (flange accommodation portion) 43 to hold the proximal end portion of the mounted syringe 2 as illustrated in Figs. 26 to 35. In other words, the syringe-mounting member 4 includes the substantially semi-tubular portion (barrel support portion) 41 that extends from the flange accommodation portion 102 in the distal direction and supports the outer peripheral surface of the tubular portion of the barrel 20. The substantially semi-tubular portion 41 is formed by a substantially semi-tubular portion that extends to the distal side from the body portion 40 (flange accommodation portion 102).

Further, the syringe-mounting member 4 includes the tubular accommodation portion 42 capable of accommodating the gasket pressing member 8 (gasket pressing assembly 6) in the proximal direction from the proximal barrel-mounting portion 43 (body portion 40). The tubular accommodation portion 42 is a tubular portion that extends rearward from the proximal end of the body portion (barrel-mounting portion) 40. The tubular accommodation portion 42 includes the accommodation portion 47 for the gasket pressing assembly therein. The tubular accommodation portion 42 extends in the proximal direction with an outer diameter smaller than an outer diameter of the proximal-side support portion 104 of the body portion (barrel-mounting portion) 40.

Further, in the syringe-mounting member 4 of the present embodiment, the tubular accommodation portion 42 has a curved portion 106 that is enlarged in diameter toward the distal end and is connected to the proximal end of the proximal-side support portion of the body portion (barrel-mounting portion) 40 as illustrated in Figs. 26 to 29. The curved portion 106 is formed in an annular shape. A radius of curvature of the curved portion 106 is preferably 0.3 to 3 mm, and particularly preferably 0.5 to 1.5 mm.

The accommodation portion 47 is formed inside the tubular accommodation portion 42. In other words, the syringe-mounting member 4 includes the tubular accommodation portion 47 capable of accommodating the gasket pressing member 8 (gasket pressing assembly 6) in the proximal direction from the proximal barrel-mounting portion 43 (body portion 40). Since such an accommodation portion 47 is provided, the gasket-mounting member 8 does not become an obstacle when the barrel 20 is mounted on the proximal barrel-mounting portion 43 of the syringe-mounting member 4.

Further, the syringe-mounting member 4 of the present embodiment includes the tubular insertion portion 110 which extends in the proximal direction of the gasket pressing tool 3 from the proximal end of the tubular accommodation portion 42 and into which the shaft portion 51 of the plunger 5 is inserted. The shaft portion 51 of the plunger 5 includes the shaft-side screwing portion 55, and the insertion-portion-side screwing portion 46 that is screwed with the shaft-side screwing portion 55 is provided on an inner peripheral surface of the insertion portion 110 (lumen 45). In other words, the insertion portion 110 (lumen 45) has the insertion-portion-side screwing portion 46 to be screwed with the shaft-side screwing portion 55 of the shaft portion 51 on its inner surface. In the present embodiment, the shaft-side screwing portion 55 is a spiral groove, and the insertion-portion-side screwing portion 46 is a spiral rib (spiral protrusion), specifically, a short spiral rib.

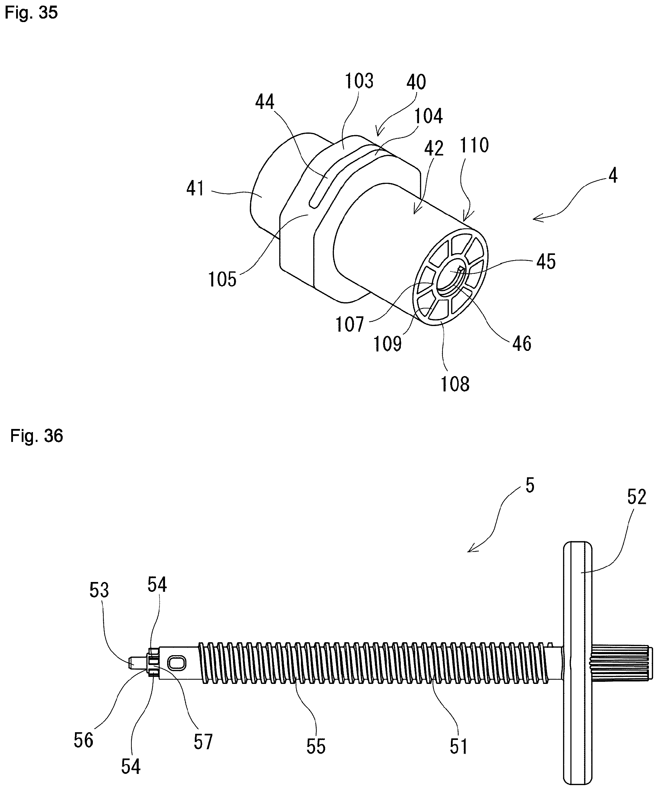

Further, as illustrated in Fig. 35, the insertion portion 110 includes: an inner tubular portion 107 including the insertion-portion-side screwing portion (short spiral rib) 46; an outer tubular portion 108 arranged concentrically with the inner tubular portion 107 on the outer side of the inner tubular portion 107 (in other words, to be separated from an outer peripheral surface of the inner tubular portion 107); and a plurality of connecting plates 109 that connect the outer peripheral surface of the inner tubular portion 107 and an inner peripheral surface of the outer tubular portion 108 and are arranged intermittently in the circumferential direction. It is preferable that four to ten connecting plates 109 be arranged at equal intervals in the circumferential direction of the syringe-mounting member 4. In addition, the connecting plate 109 extends over the entire length of the inner tubular portion 107 in the present embodiment. For this reason, the insertion portion 110, which is a site screwed with the plunger 5, is strongly reinforced, thereby preventing the syringe-mounting member 4 from being distorted at the site. In addition, the syringe-mounting member 4 of the present embodiment has a substantially uniform outer diameter from a distal end of the tubular accommodation portion 42 to a proximal end of the outer tubular portion 108 of the insertion portion 110.

As illustrated in Figs. 2 to 8, 10, and 36 to 38, the plunger 5 enters the syringe-mounting member 4 on the distal side and can press the gasket pressing assembly 6. Specifically, the plunger 5 includes the shaft portion 51 and the handle 52 provided at the proximal end portion of the shaft portion 51. The shaft portion 51 includes the shaft-side screwing portion 55 provided on the outer surface. In the present embodiment, the shaft-side screwing portion 55 is locked by the spiral groove into which the insertion-portion-side screwing portion 46 (specifically, the short spiral protrusion) of the syringe-mounting member 4 enters such that the screw can be advanced. The distal end portion of the plunger 5 advances in the syringe-mounting member when rotated to one side (rotated forward), and the distal end portion retracts in the syringe-mounting member when rotated to the other side (rotated reversely).

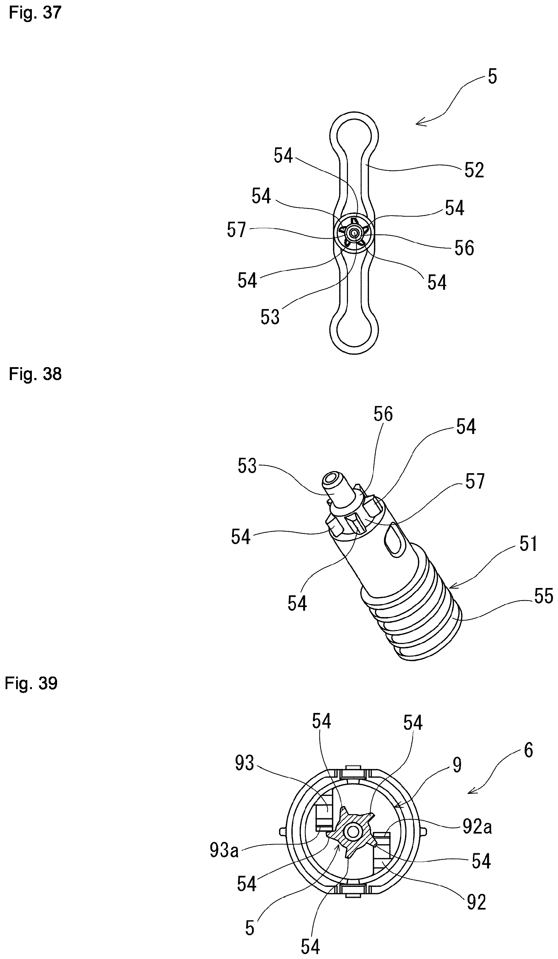

Specifically, as illustrated in Figs. 37 and 38, a distal base portion 57 that holds the claw-flicking ribs 54 on its outer surface, an annular abutment portion 56 formed by a distal surface of the distal base portion, and a small-diameter cylindrical protruding portion 53 that protrudes from the distal base portion 57 with a diameter smaller than an outer diameter of the abutment portion are provided at the distal end portion of the shaft portion 51. The cylindrical protruding portion 53 can enter the columnar recess 85 (specifically, the cylindrical recess) formed in the central portion of the gasket pressing member 8 and is rotatable after the entry.

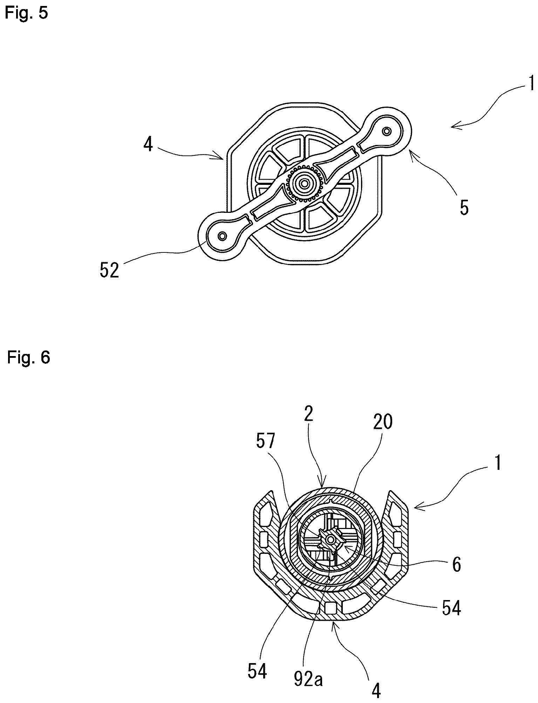

An odd number of (three of more) claw-flicking ribs 54 are provided as illustrated in Figs. 6, 38, and 39, and these claw-flicking ribs 54 are preferably arranged so as to be equiangular with respect to the central axis of the plunger 5. In the present embodiment, five claw-flicking ribs 54 are provided and arranged so as to be equiangular with respect to the central axis of the plunger 5. Further, in the present embodiment, surfaces of the claw-flicking ribs 54 that abut on the claw tip portions 92a and 93a when the plunger 5 is rotated in the screw advancing direction are formed substantially parallel to the central axis of the plunger 5 and further formed as surfaces substantially upright from an outer peripheral surface of the distal base portion 57. Further, a surface of the claw-flicking rib 54 on the side opposite to the upright surface is formed as an inclined surface. For this reason, the claw-flicking rib 54 becomes thicker downward (toward the distal base portion 57).

The handle 52 is formed at the proximal end portion of the shaft portion 51, and is formed by two protruding portions extending in the radial direction from the shaft portion 51. Further, the plunger 5 of the present embodiment has an end portion extending rearward from the handle portion 52.

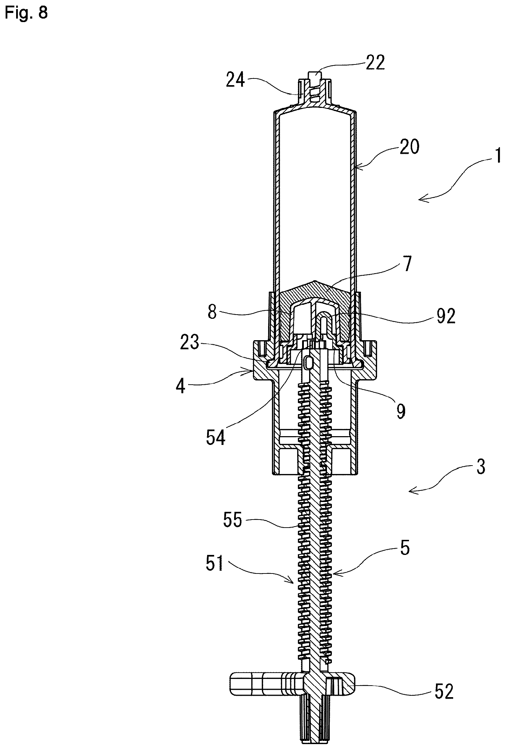

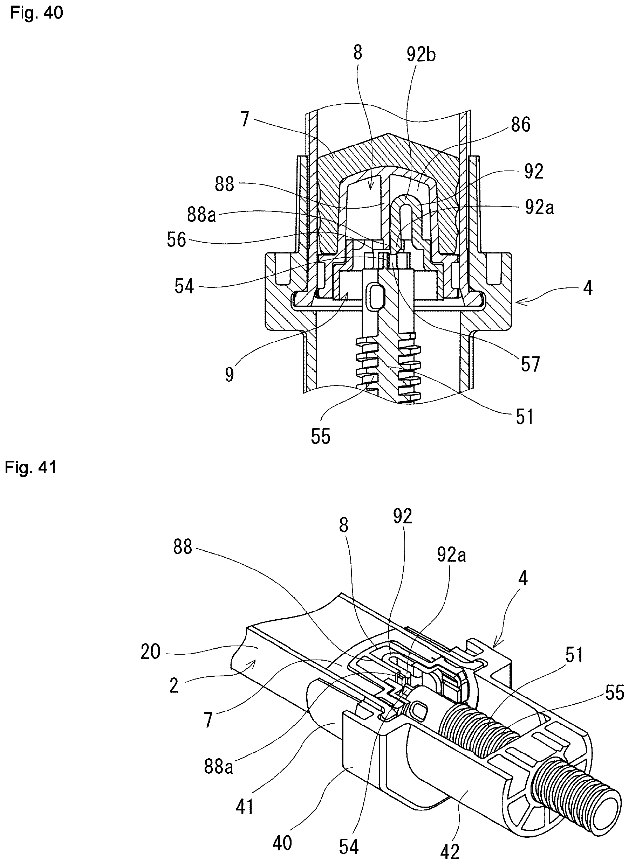

As illustrated in Fig. 8 and Fig. 40, which is an enlarged view of Fig. 8, the claw-flicking ribs 54 are located on the lateral side of the claw tip portions 92a and 93a in a state where the annular abutment portion 56 of the plunger 5 abuts on the plunger abutment portion 62 of the gasket pressing assembly 6 in a pressable manner. Further, as the plunger 5 is rotated, the plunger 5 advances to move the gasket pressing assembly 6 forward, the claw-flicking ribs 54 abut on the claw tip portions 92a and 93a and pass the claw portions 92 and 93 while deforming the claw portions 92 and 93, and further, a sound is generated with abutment of the claw portions 92 and 93 restored after the passage on the wall portions 88 and 89.

Next, operations of the liquid medicine ejection tool 1 and the liquid medicine administration tool according to the present embodiment will be described.

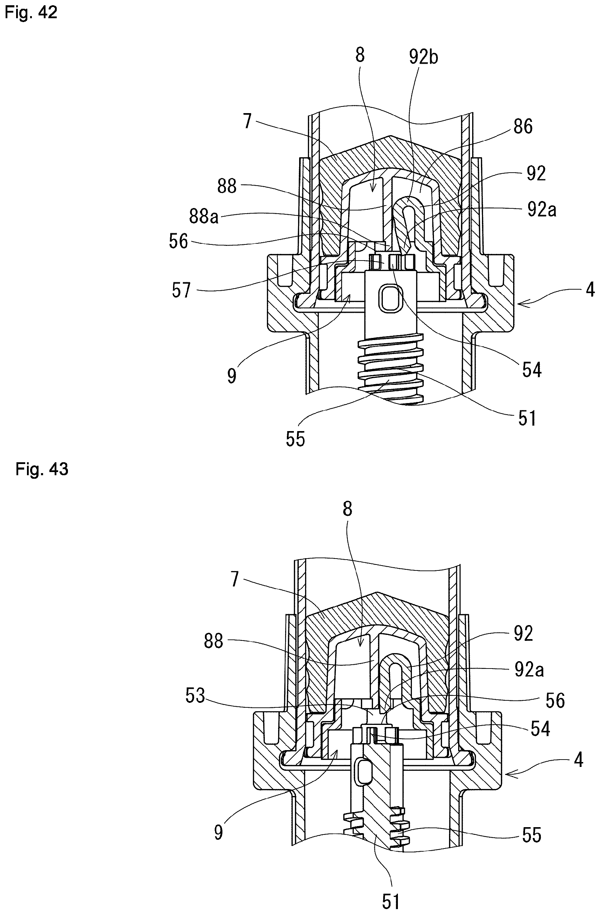

As the plunger is rotated, the state where the annular abutment portion 56 of the plunger 5 abuts on the gasket pressing assembly 6 in a pressable manner, that is, the state illustrated in Figs. 40 and 41, is formed. In this state, the claw-flicking rib 54 of the plunger 5 is located on the lateral side of each of the claw tip portions 92a and 93a. Then, as the plunger 5 is rotated, the plunger 5 advances, the gasket pressing assembly 6 also advances. As the gasket pressing assembly 6 advances, the gasket 7 also advances. Then, the claw-flicking ribs 54 of the plunger 5 abut on the claw tip portions 92a and 93a of the sound generating member 9, deform the claw portions 92 and 93, and eventually, pass through the claw portions 92 and 93 as illustrated in Fig. 42. Then, after the passage, the claw portions 92 and 93 are restored to the original shapes due to spring properties, and abut on the wall portions 88 and 89 when restored, thereby generating the sound.

In particular, the wall portion 88 is provided with a thin proximal end portion 88a in the illustrated embodiment, and the restored claw tip portion 92a abuts on the thin proximal end portion 88a. Since the thin proximal end portion is thin, a relatively high sound is generated. In addition, the two claw portions 92 and 93 and the five claw-flicking ribs 54 are provided in the present embodiment. For this reason, ten collision sounds are generated when the plunger 5 rotates. In other words, the collision sound is generated every time the plunger 5 rotates 36 degrees.

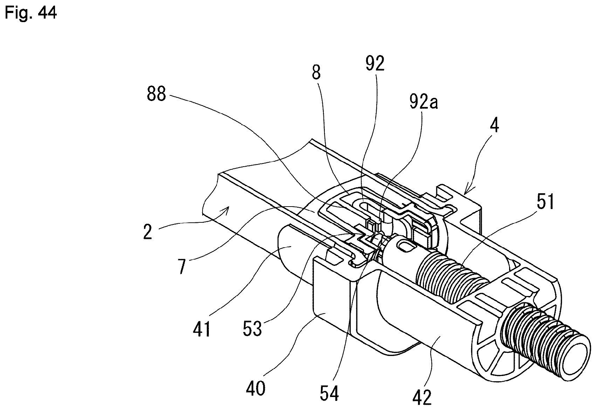

Further, in the gasket pressing tool 3 of the liquid medicine ejection tool 1 of the present embodiment, the claw-flicking rib 54 of the plunger 5 is located on the rear side of each of the claw tip portions 92a and 93a of the sound generating member 9 in the state where the abutment portion 56 of the plunger 5 does not abut on the gasket pressing assembly 6 in a pressable manner as illustrated in Figs. 43 and 44. For this reason, the claw-flicking ribs 54 do not abut on the claw tip portions 92a and 93a even if the plunger 5 is rotated, and thus, no sound is generated. In addition, the plunger 5 retracts as the plunger 5 is rotated reversely (rotated in the retracting direction) in the state illustrated in Figs. 40 and 41. No sound is generated even during this reverse rotation. Note that the liquid medicine ejection tool 1 of the present embodiment may be provided in the state where the gasket pressing assembly 6 is mounted on the gasket 7 as illustrated in Fig. 7, in other words, provided with such a state as the initial state. Note that the plunger 5 may or does not necessarily abut on the gasket pressing assembly 6.

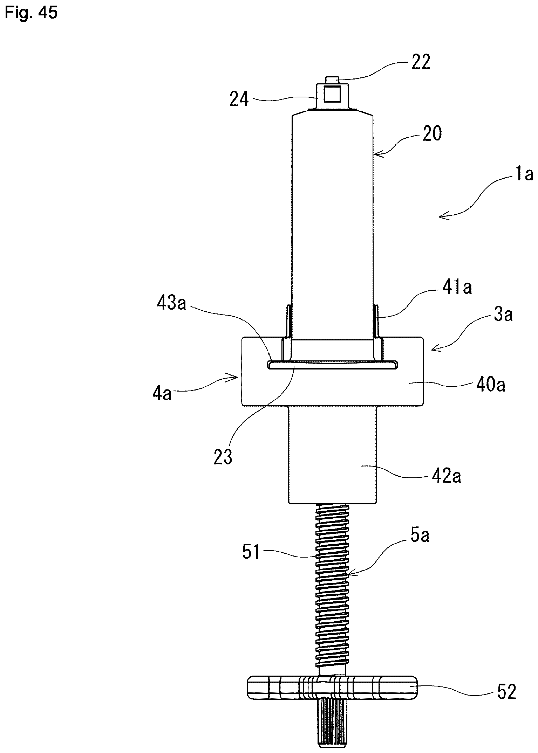

Next, a liquid medicine ejection tool 1a and a gasket pressing tool 3a of the embodiment illustrated in Figs. 45 to 47 will be described.

The liquid medicine ejection tool 1a and the gasket pressing tool 3a of the present embodiment have the same configuration regarding the sound generation as the liquid medicine ejection tool 1 and the gasket pressing tool 3 described above, and differences therebetween are a form of a syringe-mounting member and a structure of a gasket pressing assembly. The same parts are designated by the same reference signs, and the above description is referred to therefor.

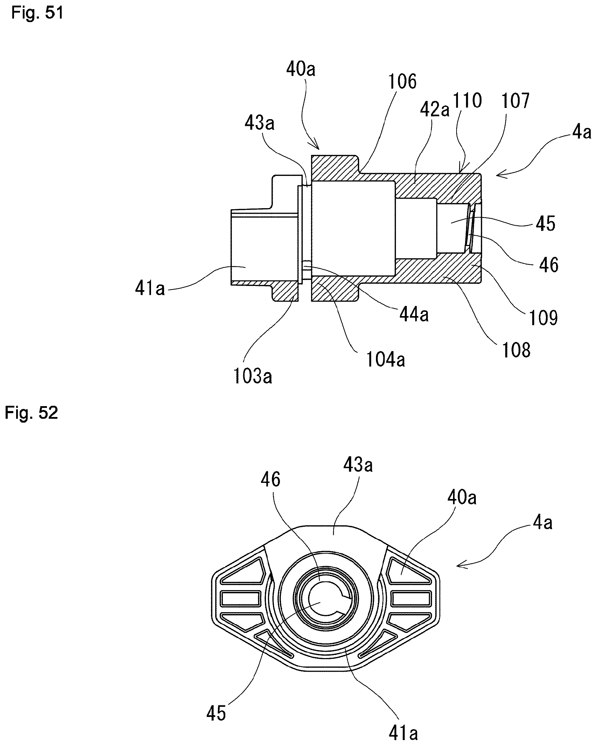

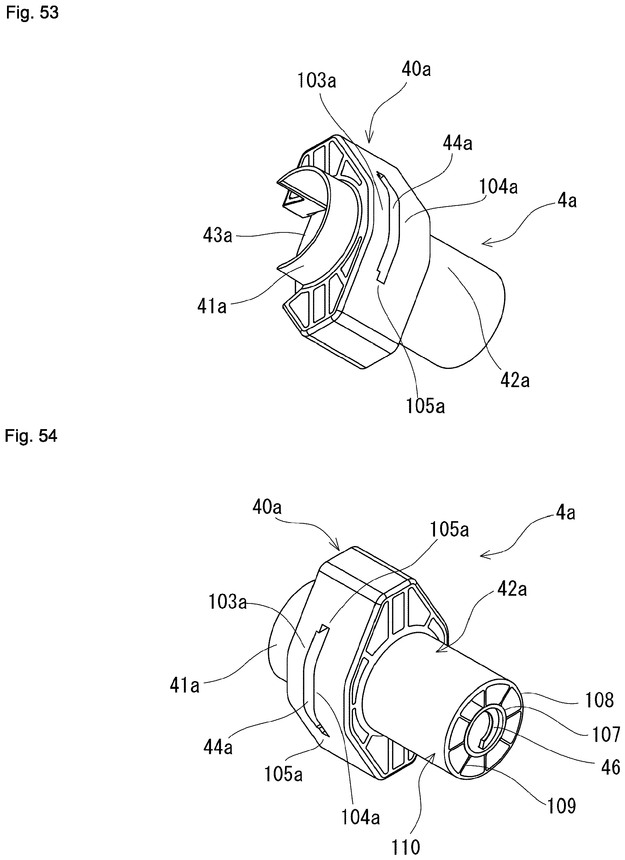

A syringe-mounting member 4a used in the gasket pressing tool 3a of the present embodiment includes a proximal barrel-mounting portion 43a as illustrated in Figs. 45 to 54. In addition, a gasket pressing assembly 6a is movably accommodated similarly to the above-described embodiment.

The syringe-mounting member 4a used in the gasket pressing tool 3a of the present embodiment includes a body portion (barrel-mounting portion) 40a as illustrated in Figs. 45 to 53. As illustrated in Figs. 45 to 54, the gasket pressing tool 3a of the present embodiment movably accommodates the gasket pressing assembly 6a similarly to the one illustrated in Fig. 2.

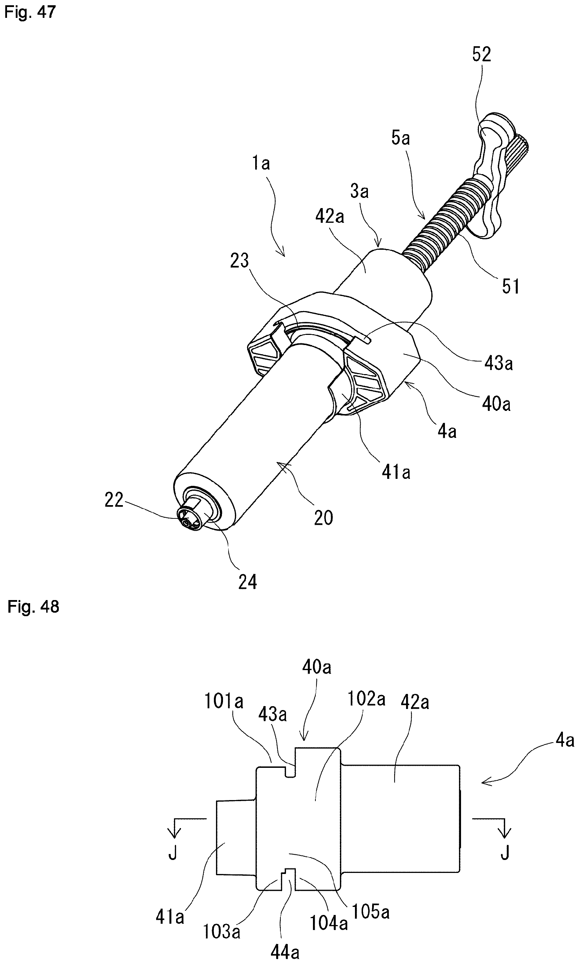

As illustrated in Figs. 47 to 54, the syringe-mounting member 4a includes: a distal-side support portion 103a, a proximal-side support portion 104a, a connecting portion 105a, a flange accommodation portion 102a, and a flange insertion opening 101a. The distal-side support portion 103a can abut on a distal surface of the flange 23 of the barrel 20 accommodated in the flange accommodation portion 102a, and supports the distal surface of the flange 23.

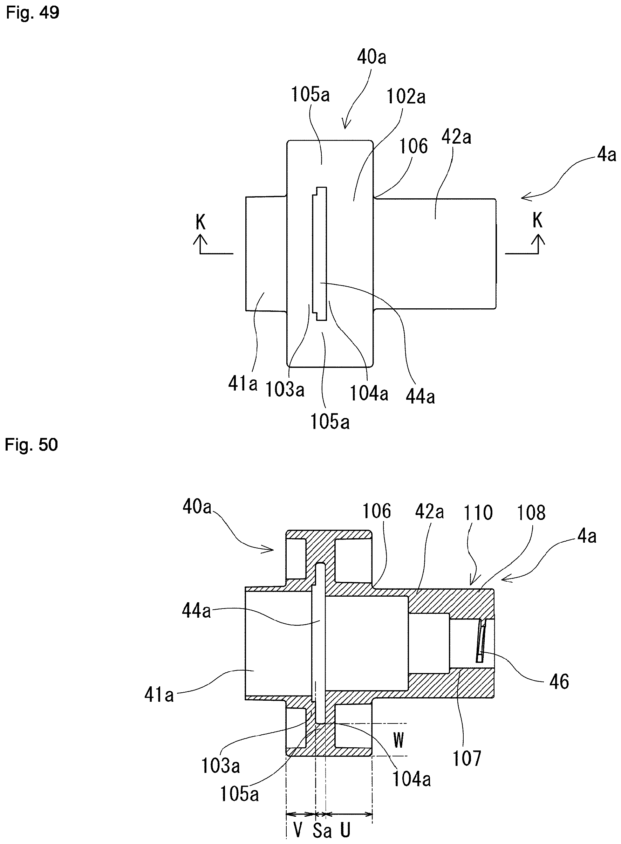

In the syringe-mounting member 4a of the present embodiment, the distal-side support portion 103a has a predetermined thickness (axial length: V) extending along the central axis of the syringe-mounting member 4a as illustrated in Fig. 50, and the thickness (V) is preferably 5 to 25 mm, and particularly preferably 10 to 15 mm. The distal-side support portion 103a can sufficiently support the distal surface of the flange. The distal-side support portion 103a is preferably capable of supporting 55 to 80% of a distal surface 23a of the flange 23 (in other words, capable of abutting on 55 to 80% of the distal surface 23a), and particularly preferably capable of supporting 70 to 80% (capable of abutting on 70 to 80%) of the distal surface 23a.

In addition, the syringe-mounting member 4a of the present embodiment, the proximal-side support portion 104a has a predetermined thickness (axial length: U) extending along the central axis of the syringe-mounting member 4a as illustrated in Fig. 50, and the thickness (U) is preferably 5 to 25 mm, and particularly preferably 10 to 15 mm. The proximal-side support portion 104a can abut on almost the entire proximal surface 23b of the flange 23. For this reason, the proximal-side support portion 104a can sufficiently support the proximal surface of the flange.

Further, the distal-side support portion 103a and the proximal-side support portion 104a are connected by the connecting portion 105a in the syringe-mounting member 4a of the present embodiment. Further, the connecting portion 105a has a predetermined thickness (axial thickness: Sa) extending along the central axis of the syringe-mounting member 4a as illustrated in Fig. 50, and the thickness (Sa) is preferably -1 to 1 mm with respect to the thickness of the flange 23 of the barrel 20, and particularly preferably -0.5 to 0.5 mm with respect to the thickness of the flange 23 of the barrel 20.

In addition, there are two connecting portions 105a facing each other in the present embodiment as illustrated in Fig. 50, and further, the connecting portion 105a has a radial width W along the radial direction of the syringe-mounting member 4a as illustrated in Fig. 50. The radial width (W) is preferably 1 to 5 mm, and particularly preferably 2 to 3 mm.

The syringe-mounting member 4a includes the proximal barrel-mounting portion 43a that accommodates the flange 23 of the barrel 20 of the syringe 2. In the present embodiment, the proximal barrel-mounting portion 43a is formed using a slit to be capable of inserting the flange 23 of the syringe 2 provided in the body portion 40a from one short-diameter side (in other words, allow horizontal insertion) as illustrated in Figs. 47 and 50. For this reason, in the state where the syringe 2 is mounted on the syringe-mounting member 4a, one short-diameter portion of the flange 23 and two long-diameter portions facing each other of the syringe 2 are accommodated in the syringe-mounting member 4a, and a short-diameter portion on the other end side is exposed. In addition, an end portion of one short-diameter portion of the flange accommodated in the syringe-mounting member 4a is exposed through a slit opening 44a. In the present embodiment, the proximal barrel-mounting portion 43a is formed by a horizontally elongated U-shaped slit.

In addition, in the present embodiment, the syringe-mounting member 4a includes: a substantially semi-tubular portion (barrel support portion) 41a extending to the distal side from the proximal barrel-mounting portion (flange accommodation portion) 43a to hold the proximal end portion of the mounted syringe 2 as illustrated in Figs. 48 to 51. Further, the syringe-mounting member 4a includes a tubular accommodation portion 42a extending from the proximal barrel-mounting portion (flange accommodation portion) 43a to the proximal side. The tubular accommodation portion 42a includes the accommodation portion 47 for the gasket pressing assembly therein. Further, the syringe-mounting member 4a includes the lumen 45 extending rearward from the accommodation portion 47, and a protrusion (insertion-portion-side screwing portion) 46 is provided on an inner surface of the lumen 45. The protrusion 46 is a short spiral protrusion.

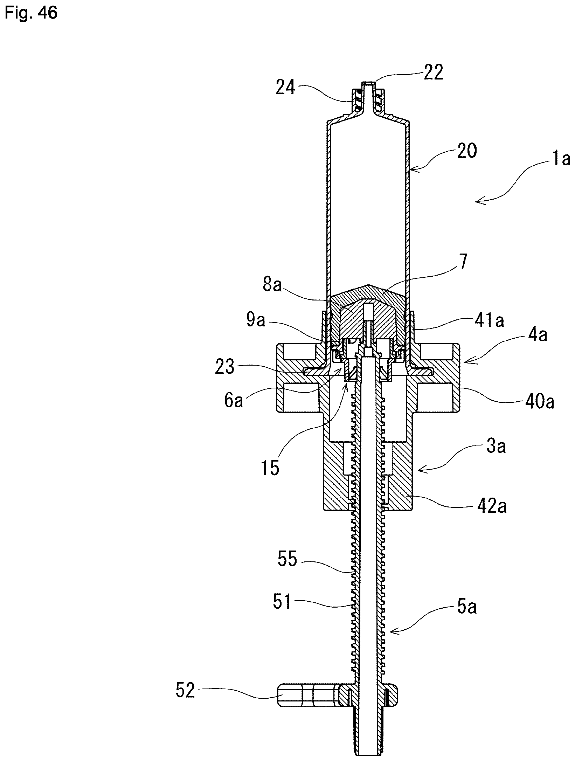

As illustrated in Figs. 46, 55, and 56, the gasket pressing assembly 6a of the present embodiment includes: a gasket pressing member 8a, a sound generating member 9a, and a joint member 15. As illustrated in Figs. 46, 55, and 56, the gasket pressing member 8a includes: the gasket-mounting portion 80 that enters the gasket 7 and comes into contact with the inner surface of the gasket; the gasket proximal surface pressing portion 81 formed at a rear portion of the gasket-mounting portion 80; an annular portion 82a extending rearward from the gasket proximal surface pressing portion 81; and an annular rib 66 formed at a proximal end portion of the annular portion 82a. The annular rib 66 is provided with a notch 67. The gasket-mounting portion 80 includes a distal closing portion and a tapered diameter-reducing portion extending from the gasket proximal surface pressing portion 81 to a proximal end of the distal closing portion.

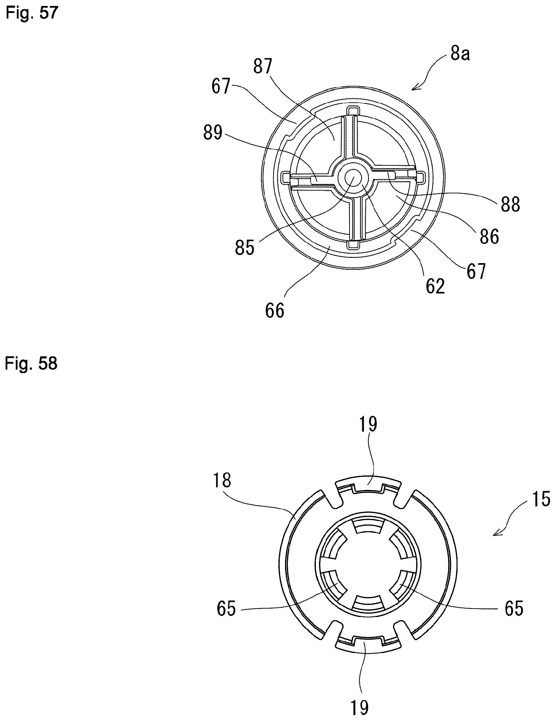

Further, as illustrated in Fig. 57, the gasket pressing member 8a of the present embodiment also includes a sound generating member accommodation portion, the wall portions 88 and 89 extending in the axial direction, the plunger abutment portion 62, and the columnar recess 85 therein and includes a cylindrical portion having the columnar recess 85 and four partition portions extending to the lateral side from the cylindrical portion at the center, which is similar to the above-described embodiment. The internal form of the gasket pressing member 8a is the same as that of the gasket pressing member 8 described above.

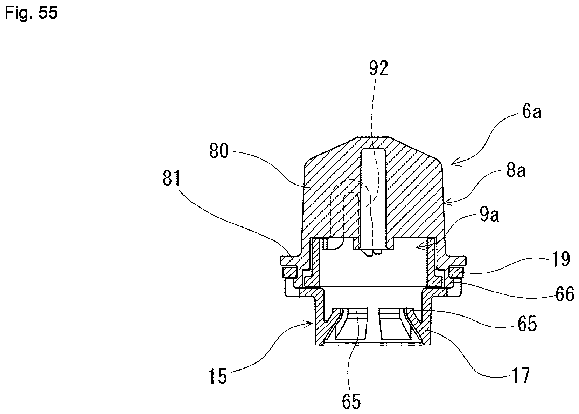

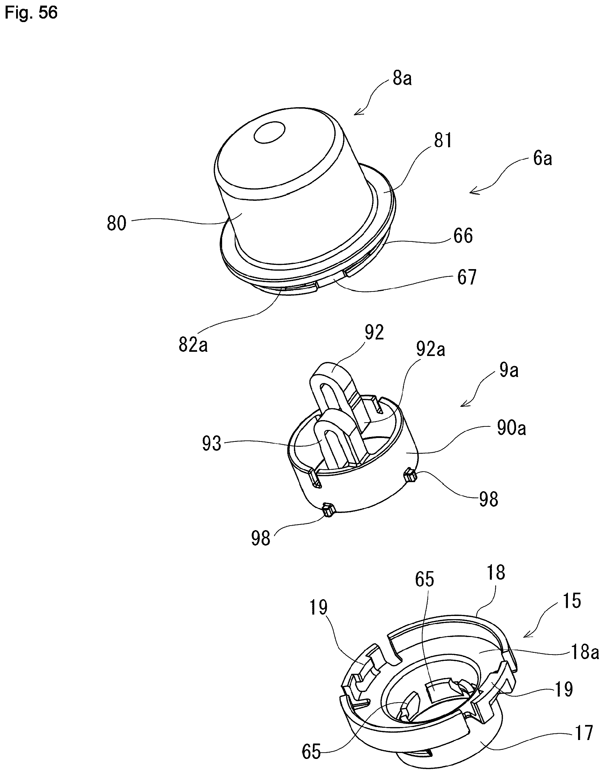

The sound generating member 9a of the present embodiment includes: a tubular body portion 90a; the claw portions 92 and 93 protruding in the distal direction from an inner surface of the tubular body portion 90a; a plurality of protruding portions 98 provided on a lower side surface of the tubular body portion 90a as illustrated in Figs. 55 and 56. The claw portions 92 and 93 have the same form as that of the sound generating member 9 described above.

In the gasket pressing member 8a of the present embodiment, as described above, the gasket pressing member 8a has the same internal form as the gasket pressing member 8 described above, the sound generating member 9a also has the same form as the sound generating member 9 described above, and a sound generating mechanism in the gasket pressing member 8a of the present embodiment is the same as the one described above.

The gasket pressing assembly 6a of the present embodiment includes the joint member 15 arranged at a rear portion of the gasket pressing member 8a. The joint member 15 is mounted on the rear portion of the gasket pressing member 8a that is open and accommodates the sound generating member 9a. The joint member 15 includes a short cylindrical portion 18 having a disc-shaped bottom surface 18a and a small-diameter cylindrical portion 17 extending rearward from the cylindrical portion 18. A rib 19 protruding inward is provided at an upper end of the cylindrical portion 18. The joint member 15 is attached to the gasket pressing member 8a as illustrated in Fig. 55 by being rotated after the rib 19 is inserted into the notch 67 of the annular rib 66 formed at the proximal end of the gasket pressing member 8a. In addition, the joint member 15 includes a plurality of inclined protruding portions 65 extending from an inner surface of the small-diameter cylindrical portion 17 toward the center of the joint member in the distal direction. The plurality of inclined protruding portions 65 are arranged so as to be equiangular with respect to a central axis of the joint member 15.

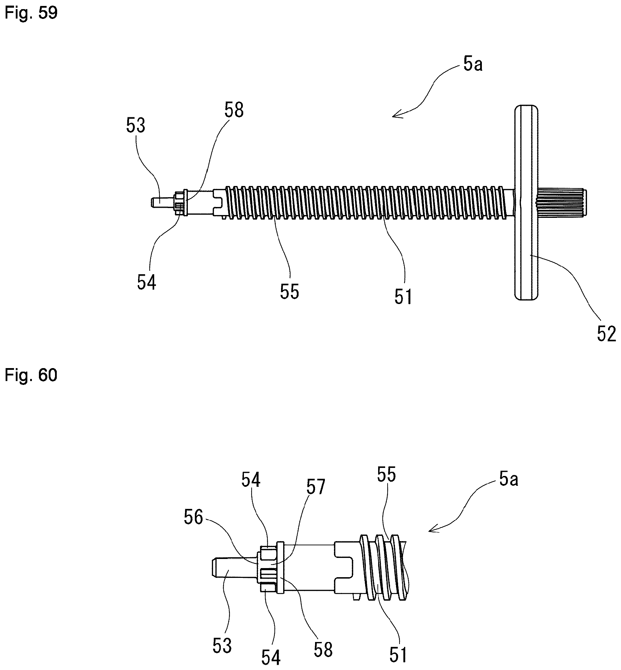

As illustrated in Figs. 59 and 60, the plunger 5a of the present embodiment has an annular rib 58 which is a distal end portion and is formed on the proximal side of the claw-flicking rib 54. An inner diameter of a portion having the minimum inner diameter, formed by the plurality of inclined protruding portions 65 of the joint member 15, is slightly larger than an outer diameter of the annular rib 58 of the plunger 5a. For this reason, the joint member 15 has a function of preventing detachment of the plunger 5a. In addition, the plunger 5a of the present embodiment includes the claw-flicking ribs 54 and has the same configuration of the distal end portion as the plunger 5 described above. The claw-flicking ribs 54 is located on the lateral side of the claw tip portions 92a and 93a in a state where the annular abutment portion 56 of the plunger 5a abuts on the gasket pressing assembly 6a in a pressable manner. Further, as the plunger 5a is rotated, the plunger 5a advances to move the gasket pressing assembly 6a forward, the claw-flicking ribs 54 abut on the claw tip portions 92a and 93a and pass the claw portions 92 and 93 while deforming the claw portions 92 and 93, and further, a sound is generated with abutment of the claw portions 92 and 93 restored after the passage on the wall portions 88 and 89.

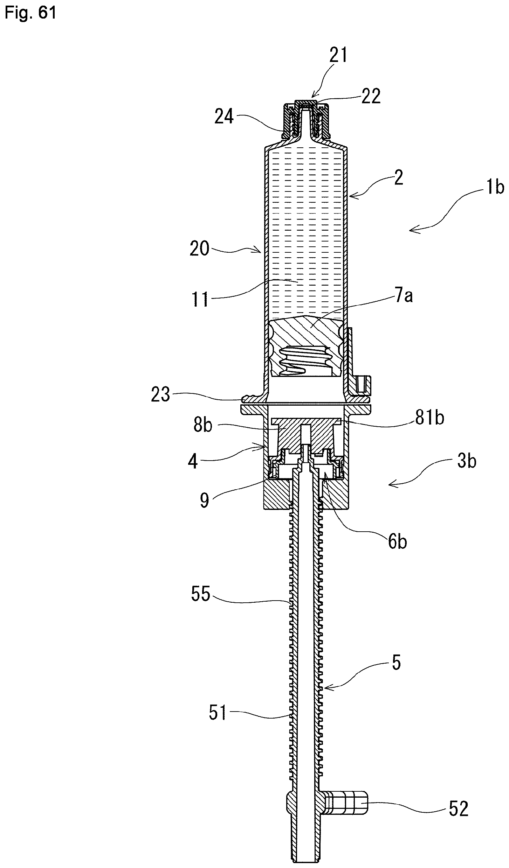

Note that a gasket pressing member may be of a type including a liquid medicine administration tool 1b and a gasket pressing tool 3b for a syringe according to an embodiment illustrated in Fig. 61 in part of the above-described embodiments. A gasket pressing member 8b of the present embodiment does not practically have a portion that enters the gasket 7a. For this reason, a gasket pressing assembly 6b also does not practically have a portion that enters the gasket 7a. Further, the gasket pressing member 8b includes a pressing portion 81b, which can abut on and press a rear end surface of the gasket 7a, at its distal end portion. Note that the liquid medicine administration tool 1b and the gasket pressing tool 3b for a syringe according to the present embodiment also have the same sound generating mechanism as the liquid medicine administration tools 1 and 1a and the gasket pressing tools 3 and 3a for the syringe described above.

Next, a case of performing endoscopic submucosal dissection (ESD) or endoscopic mucosal resection (EMR) will be described as an example of a treatment method using the liquid medicine administration tool (liquid medicine ejection tool 1) of the present invention. First, the endoscopic submucosal dissection will be described. This procedure is usually performed by two operators.

A first operator injects a sodium hyaluronate solution, and a second operator performs the excision of an affected site for confirmation of an endoscopic image. The first operator prepares the liquid medicine administration tool (liquid medicine ejection tool 1) in which the gasket pressing tool 3 is mounted on the prefilled syringe 2 filled with a sodium hyaluronate solution 11. When the prepared liquid medicine administration tool is in the state illustrated in Figs. 1 and 2, the plunger 5 is rotated to mount the gasket pressing assembly 6 in the gasket 7 as illustrated in Fig. 7. Then, the sealing cap 21 is removed from the prefilled syringe 2.

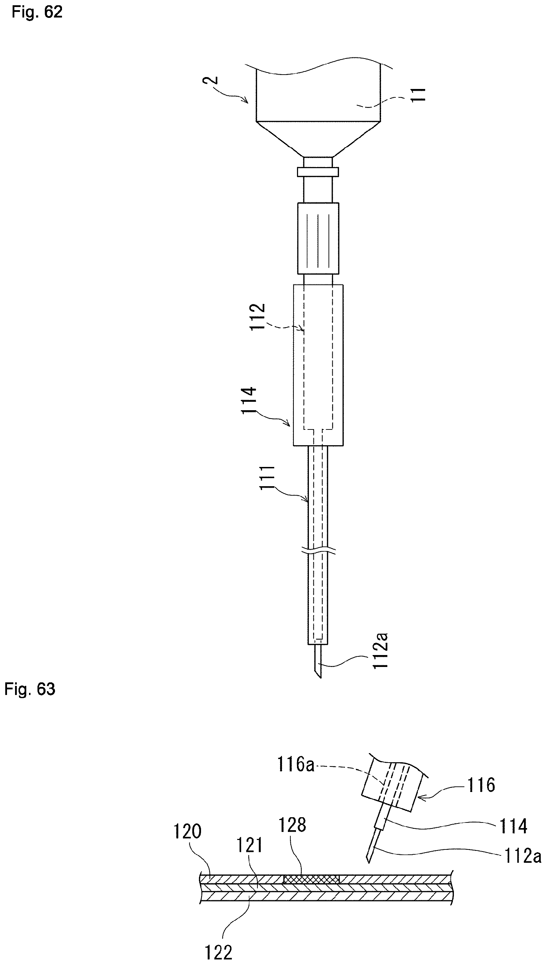

Next, as illustrated in Fig. 62, the first operator connects a distal end portion of the syringe 2 to a proximal end portion of an inner catheter 112 of a puncture device 111 and causes a needle 112a to protrude from an outer catheter 114. In this state, the plunger 5 is rotated to perform priming to fill the inner catheter 112 and a lumen of the needle 112a with the sodium hyaluronate solution 11. Note that the inner catheter 112 of the puncture device 111 is slidable in the axial direction within a regulated range with respect to the outer catheter 114. The needle 112a is connected to a distal end of the inner catheter 112. When the inner catheter 112 is at a retracted position relative to the outer catheter 114, the needle 112a is accommodated in the outer catheter 114. When the inner catheter 112 is at an advanced position relative to the outer catheter 144, the needle 112a protrudes from a distal end of the outer catheter 114.

Next, the second operator accommodates the needle 112a in the outer catheter 114. Then, the puncture device 111 is inserted into an insertion hole (forceps hole) 116a (see Fig. 63) of an endoscope 116. Subsequently, the second operator causes the needle 112a to protrude from a distal end of the endoscope 116 in a digestive tract of a patient as illustrated in Fig. 63. Then, the needle 112a penetrates a mucosa 120 around a lesion 128 to reach a submucosa 121. The second operator informs the first operator that the needle has reached a mucosal layer 121.

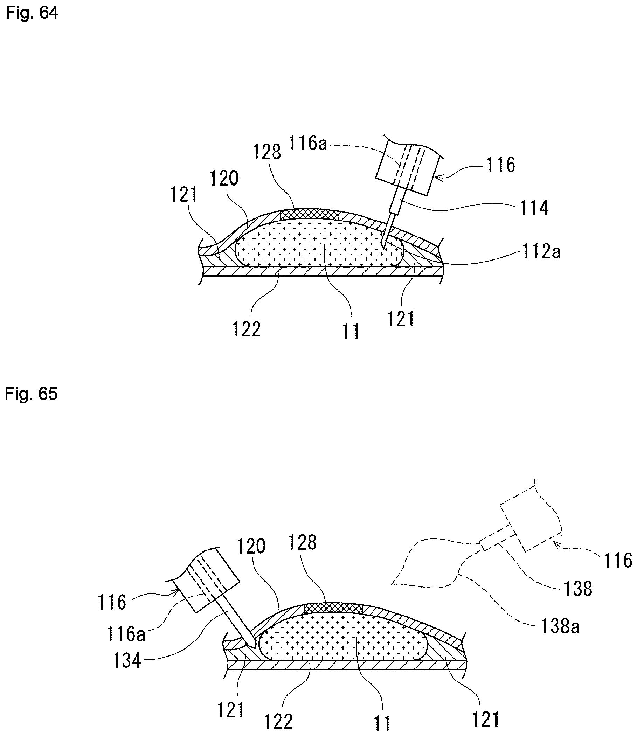

The second operator informs the first operator of the number of click sounds for injection. The first operator rotates the plunger 5 by the number of informed click sounds to inject the sodium hyaluronate solution 11 into the submucosa 121. The second operator visually confirms that a predetermined amount of the sodium hyaluronate solution 11 has been injected by confirming the number of click sounds. The second operator confirms the endoscopic image and, if necessary, informs the first operator of the number of click sounds for the maximum injection. The first operator rotates the plunger 5 by the number of informed click sounds to inject the sodium hyaluronate solution 11 into the submucosa 121. As this operation is performed a required number of times to raise the lesion 128 to a target size, the state illustrated in Fig. 64 is obtained. In this state, the submucosa 121 below the lesion 128 is turned into a state of being separated from a muscle layer 122.

The second operator notifies the first operator that the injection operation of the sodium hyaluronate solution has been completed. The first operator who has been notified reversely rotates the plunger 5 to release the pressure. As a result, the ejection of the sodium hyaluronate solution 11 from the syringe 2 is stopped.

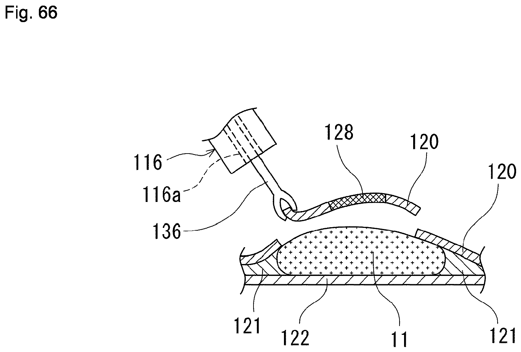

Next, the second operator inserts a peeling device 134 (needle knife or the like) into the insertion hole (forceps hole) 116a of the endoscope 116 as illustrated in Fig. 65 to cut the mucosa 120 around the lesion 128 using the peeling device 134. Subsequently, the second operator inserts a grasping device 136 into the insertion hole 116a of the endoscope 116 and peels the mucosa 120 having the lesion 128 and taken the peeled mucosa 120 out of the body using the grasping device 136 as illustrated in Fig. 66.

On the other hand, in the case of performing the endoscopic mucosal resection, a cutting device 138 illustrated by the virtual line in Fig. 65 is used, instead of peeling the mucosa 120 with the peeling device 134 described above. The cutting device 138 has a snare ring 138a (loop-shaped electric knife). The second operator causes the snare ring 138a to protrude from the endoscope 116 inside the patient's body and hooks the snare ring 138a onto the mucosa 120 around the raised lesion 128. Then, the snare ring 138a is narrowed, a high-frequency current is caused to flow through the snare ring 138a so that the mucosa 120 having the lesion 128 is cut. The cut mucosa 120 is collected by the grasping device 136 illustrated in Fig. 66. Other procedure steps are performed in the same manner as the above-described endoscopic submucosal dissection.

## Claims

1. A liquid medicine ejection tool (1) comprising: a syringe (2) that includes a barrel (20) having a flange (23) at a proximal end portion and a gasket (7) slidable inside the barrel (20) and made of an elastic material; and a gasket pressing tool (3) used by being mounted on the syringe (2), wherein
the gasket pressing tool (3) includes: a gasket pressing member (8) having a gasket-mounting portion (80), which is capable of entering the lumen portion (70) of the gasket (7) and made of a material harder than the gasket (7), and a gasket proximal surface pressing portion (81) provided at a proximal end portion of the gasket-mounting portion (80); a syringe-mounting member (4) that movably accommodates the gasket pressing member (8) and has a proximal barrel-mounting portion that is mountable to a proximal end portion of the barrel (20) having the flange (23); and a plunger (5) that has a distal end portion entering the syringe-mounting member (4) and is capable of pressing the gasket pressing member (8) in a distal direction,
**characterized in that**
the gasket (7) includes a tubular portion (72), a distal closing portion (71) that closes a distal end of the tubular portion (72), a lumen portion (70) defined by the tubular portion (72) and the distal closing portion (71), and an annular rib (73, 74, 75) provided on an outer surface of the tubular portion (72),
the gasket (7) has an original lumen portion axial length S when the gasket (7) is not inserted into the barrel (20) and the gasket pressing member (8) is not mounted, the gasket-mounting portion (80) of the gasket pressing member (8) has an axial length T, and the axial length T is longer than the original lumen portion axial length S, and
when the gasket (7) is inserted into the barrel (20) and the gasket pressing member (8) is mounted, a mounting portion distal surface of the gasket-mounting portion (80) of the gasket pressing member (8) abuts on a lumen distal surface (76) of the lumen portion (70) of the gasket (7), and the gasket proximal surface pressing portion (81) of the gasket pressing member (8) becomes close to or abuts on a proximal surface of the gasket (7).

2. The liquid medicine ejection tool (1) according to claim 1, wherein
the axial length T is 1.05 to 1.20 times of the original lumen portion axial length S.

3. The liquid medicine ejection tool (1) according to claim 1 or 2, wherein the gasket-mounting portion (80) includes: a distal end portion having a distal surface (80a) of the mounting portion; and a proximal-side tapered portion (80c) which extends from the distal end portion of the gasket-mounting portion (80) to the gasket proximal surface pressing portion (81) and is enlarged in diameter toward a rear side to have a tapered shape, and a whole outer surface of the proximal-side tapered portion (80c) becomes close to or abuts on an inner surface of the lumen portion (70) of the gasket (7).

4. The liquid medicine ejection tool (1) according to claim 3, wherein
a taper angle of the proximal-side tapered portion of the gasket-mounting portion (80) is 1 to 8 degrees.

5. The liquid medicine ejection tool (1) according to any one of claims 1 to 4, wherein a whole outer surface of the gasket-mounting portion (80) has a shape that substantially coincides with a whole inner surface of the lumen portion (70) of the gasket (7) when being inserted into the barrel (20).

6. The liquid medicine ejection tool (1) according to any one of claims 1 to 5, wherein
the lumen distal surface (76) of the lumen portion (70) of the gasket (7) has: a flat central flat surface at a center of the lumen distal surface (76); and a lumen portion tapered surface (76b) that extends rearward and radially outward from an outer edge of the central flat surface,
the distal closing portion (71) of the gasket (7) has: a tapered gasket distal surface (71a) whose diameter is reduced toward a distal end; and a uniform-thickness portion (71b) which is sandwiched between the gasket distal surface (71a) and the lumen portion tapered surface and has a substantially uniform thickness,
the mounting portion distal surface (80a) of the gasket-mounting portion (80) has: a flat distal surface, which is flat, at a center of the mounting portion distal surface (80a); and a tapered outer edge surface extending rearward and radially outward from an outer edge of the flat distal surface, and
when the gasket (7) is inserted into the barrel (20) and the gasket pressing member (8) is mounted, the flat distal surface of the gasket-mounting portion (80) abuts on the central flat surface of the lumen portion (70) of the gasket (7), and the tapered outer edge surface of the gasket-mounting portion (80) becomes close to or abuts on the lumen portion tapered surface of the lumen portion (70) of the gasket (7).

7. The liquid medicine ejection tool (1) according to any one of claims 1 to 6, wherein the gasket (7) has an original axial length Q and an original lumen portion axial length S in the state where the gasket (7) is not inserted into the syringe (2) and the gasket pressing member (8) is not mounted on, the gasket (7) has a deformed axial length P and a deformed lumen portion axial length R in the state where the gasket (7) is inserted into the syringe (2) and the gasket pressing member (8) is mounted, the deformed axial length P is longer than the original axial length Q, and the deformed lumen portion axial length R is longer than the original lumen portion axial length S.

8. The liquid medicine ejection tool (1) according to any one of claims 1 to 7, wherein the gasket pressing member (8) includes a plunger abutment portion (62) that is capable of abutting on and being separated from the distal end portion of the plunger (5).

9. The liquid medicine ejection tool (1) according to claim 8, wherein in the state where the gasket (7) is inserted into the barrel (20) and the gasket pressing member (8) is mounted, the distal end portion of the plunger (5) abuts on the plunger abutment portion (62) of the gasket pressing member (8) when the plunger (5) is advanced in the distal direction, and the mounting state of the gasket pressing member (8) on the gasket (7) is maintained and the plunger abutment (62) portion is separated from the distal end portion of the plunger (5) when the plunger (5) is pulled in a proximal direction.

10. The liquid medicine ejection tool (1) according to claim 9, wherein when the plunger (5) is pulled in the proximal direction in the state where the gasket (7) is inserted into the barrel (20) and the gasket-mounting portion (80) is inserted into the lumen portion (70) of the gasket (7), the mounting portion distal surface (80a) of the gasket-mounting portion (80) maintains a state of abutting on the lumen distal surface of the lumen portion (70) of the gasket (7).

11. The liquid medicine ejection tool (1) according to any one of claims 1 to 10, wherein the syringe-mounting member (4) includes a tubular accommodation portion (42) that extends in the proximal direction from a body portion having the proximal barrel-mounting portion and is capable of accommodating the gasket-mounting portion (80).

12. The liquid medicine ejection tool (1) according to claim 11, wherein
the plunger (5) includes: a shaft portion (51) which extends in the proximal direction from the distal end portion and has a shaft-side screwing portion (55) on an outer peripheral surface; and a handle (52) configured to rotate the plunger (5) provided at a proximal end portion of the shaft portion,
the syringe-mounting member (4) includes a tubular insertion portion (110) which extends in the proximal direction from the tubular accommodation portion (42) and in which the shaft portion of the plunger (5) is inserted,
the insertion portion has an insertion-portion-side screwing portion (46), which is screwed with the shaft-side screwing portion (55) of the shaft portion, on an inner surface, and
the plunger (5) is advanced in the distal direction by screwing between the shaft-side screwing portion (55) and the insertion-portion-side screwing portion (46)along with rotation of the shaft portion.

13. A liquid medicine administration tool comprising: the liquid medicine ejection tool (1) according to any one of claims 1 to 12; a liquid medicine charged in the syringe (2); and a sealing member (21) sealing the distal end portion of the barrel (20).

## Patentansprüche

1. Ausstoßwerkzeug (1) zur Abgabe eines flüssigen Arzneimittels, mit: einer Spritze (2), die einen Zylinder (20) mit einem Flansch (23) an einem proximalen Endabschnitt und eine Dichtung (7) hat, die innerhalb des Zylinders (20) verschiebbar ist und aus einem elastischen Material hergestellt ist; und einem Werkzeug (3) zum Pressen der Dichtung, das an der Spritze (2) montiert verwendet wird, wobei
das Werkzeug (3) zum Pressen der Dichtung folgendes hat: ein Dichtungspresselement (8) mit einem Dichtungsbefestigungsabschnitt (80), der in der Lage ist, in den Lumenabschnitt (70) der Dichtung (7) einzudringen, und der aus einem härteren Material als die Dichtung (7) hergestellt ist, und einem proximalen Dichtungsflächenpressabschnitt (81), der an einem proximalen Endabschnitt des Dichtungsbefestigungsabschnitts (80) bereitgestellt ist; ein Spritzenbefestigungselement (4), das das Dichtungsdruckelement (8) beweglich aufnimmt und einen proximalen Zylinderbefestigungsabschnitt aufweist, der an einem proximalen Endabschnitt des Zylinders (20) mit dem Flansch (23) befestigt werden kann; und einen Kolben (5), der einen distalen Endabschnitt aufweist, der in das Spritzenbefestigungselement (4) eintritt und in der Lage ist, das Dichtungsdruckelement (8) in einer distalen Richtung zu drücken,
**dadurch gekennzeichnet, dass**
die Dichtung (7) einen röhrenförmigen Abschnitt (72), einen distalen Verschlussabschnitt (71), der ein distales Ende des röhrenförmigen Abschnitts (72) verschließt, einen Lumenabschnitt (70), der durch den röhrenförmigen Abschnitt (72) und den distalen Verschlussabschnitt (71) definiert ist, und eine ringförmige Rippe (73, 74, 75) hat, die an einer Außenfläche des röhrenförmigen Abschnitts (72) bereitgestellt ist,
die Dichtung (7) eine ursprüngliche axiale Länge S des Lumenabschnitts aufweist, wenn die Dichtung (7) nicht in den Zylinder (20) eingesetzt ist und das Dichtungspresselement (8) nicht montiert ist, der Dichtungsmontageabschnitt (80) des Dichtungspresselements (8) eine axiale Länge T aufweist und die axiale Länge T größer als die ursprüngliche axiale Länge S des Lumenabschnitts ist, und
wenn die Dichtung (7) in den Zylinder (20) eingesetzt ist und das Dichtungspresselement (8) montiert ist, eine distale Oberfläche des Montageabschnitts des Dichtungsmontageabschnitts (80) des Dichtungspresselements (8) an einer distalen Lumenoberfläche (76) des Lumenabschnitts (70) der Dichtung (7) anliegt, und der proximale Oberflächenpressabschnitt (81) der Dichtung des Dichtungspresselements (8) sich einer proximalen Oberfläche der Dichtung (7) nähert oder an dieser anliegt.

2. Ausstoßwerkzeug (1) zur Abgabe eines flüssigen Arzneimittels gemäß Anspruch 1, wobei die axiale Länge T das 1,05 - bis 1,20 -fache der ursprünglichen axialen Länge S des Lumenabschnitts beträgt.

3. Ausstoßwerkzeug (1) zur Abgabe eines flüssigen Arzneimittels gemäß Anspruch 1 oder 2, wobei der Abschnitt (80) zur Befestigung der Dichtung Folgendes hat:
einen distalen Endabschnitt mit einer distalen Oberfläche (80a) des Befestigungsabschnitts; und einen proximalseitigen abgeschrägten Abschnitt (80c), der sich von dem distalen Endabschnitt des Dichtungsbefestigungsabschnitts (80) zu dem proximalen Oberflächendruckabschnitt (81) der Dichtung erstreckt und im Durchmesser zu einer Rückseite hin vergrößert ist, um eine abgeschrägte Form zu haben, und eine gesamte Außenfläche des proximalseitigen abgeschrägten Abschnitts (80c) nahe an eine Innenfläche des Lumenabschnitts (70) der Dichtung (7) herankommt oder daran anstößt.

4. Ausstoßwerkzeug (1) zur Abgabe eines flüssigen Arzneimittels gemäß Anspruch 3, wobei der Schrägungswinkel des proximalseitigen abgeschrägten Abschnitts des Dichtungsbefestigungsabschnitts (80) 1 bis 8 Grad beträgt.

5. Ausstoßwerkzeug (1) zur Abgabe eines flüssigen Arzneimittels gemäß einem der Ansprüche 1 bis 4, wobei eine gesamte äußere Oberfläche des Abschnitts (80) zur Befestigung der Dichtung eine Form hat, die im Wesentlichen mit einer gesamten inneren Oberfläche des Lumenabschnitts (70) der Dichtung (7) übereinstimmt, wenn sie in den Zylinder (20) eingeführt wird.

6. Ausstoßwerkzeug (1) zur Abgabe eines flüssigen Arzneimittels gemäß einem der Ansprüche 1 bis 5, wobei
die distale Lumenfläche (76) des Lumenabschnitts (70) der Dichtung (7) aufweist: eine flache zentrale flache Fläche in einem Mittelpunkt der distalen Lumenfläche (76); und eine abgeschrägte Lumenabschnittsfläche (76b), die sich von einem äußeren Rand der zentralen flachen Fläche nach hinten und radial nach außen erstreckt,
der distale Schließabschnitt (71) der Dichtung (7) hat: eine abgeschrägte distale Dichtungsfläche (71a), deren Durchmesser zu einem distalen Ende hin abnimmt; und einen Abschnitt (71b) mit gleichmäßiger Dicke, der zwischen der distalen Dichtungsfläche (71a) und der abgeschrägten Fläche des Lumenabschnitts liegt und eine im Wesentlichen gleichmäßige Dicke hat,
die distale Oberfläche (80a) des Abschnitts (80) zur Befestigung der Dichtung hat: eine flache distale Oberfläche, die flach ist, in einem Mittelpunkt der distalen Oberfläche (80a) des Abschnitts zur Befestigung; und eine abgeschrägte äußere Kantenoberfläche, die sich von einer äußeren Kante der flachen distalen Oberfläche nach hinten und radial nach außen erstreckt, und
wenn die Dichtung (7) in den Zylinder (20) eingesetzt und das Dichtungspresselement (8) montiert wird, die flache distale Oberfläche des Dichtungsmontageabschnitts (80) an der zentralen flachen Oberfläche des lumenförmigen Abschnitts (70) der Dichtung (7) anliegt und die abgeschrägte äußere Randoberfläche des Dichtungsmontageabschnitts (80) nahe an die abgeschrägte Oberfläche des lumenförmigen Abschnitts (70) der Dichtung (7) herankommt oder daran anliegt.

7. Ausstoßwerkzeug (1) zur Abgabe eines flüssigen Arzneimittels gemäß einem der Ansprüche 1 bis 6, wobei die Dichtung (7) eine ursprüngliche axiale Länge Q und eine ursprüngliche axiale Länge S des Lumenabschnitts in dem Zustand aufweist, in dem die Dichtung (7) nicht in die Spritze (2) eingesetzt ist und das Dichtungsanpresselement (8) nicht aufgesetzt ist, die Dichtung (7) eine verformte axiale Länge P und eine verformte axiale Länge R des Lumenabschnitts in dem Zustand aufweist, in dem die Dichtung (7) in die Spritze (2) eingesetzt ist und das Dichtungsandrückelement (8) montiert ist, die verformte axiale Länge P länger als die ursprüngliche axiale Länge Q ist und die verformte axiale Länge R des Lumenabschnitts länger als die ursprüngliche axiale Länge S des Lumenabschnitts ist.

8. Ausstoßwerkzeug (1) zur Abgabe eines flüssigen Arzneimittels gemäß einem der Ansprüche 1 bis 7, wobei das Dichtungspresselement (8) einen Kolbenanschlagabschnitt (62) hat, der an dem distalen Endabschnitt des Kolbens (5) anliegen und von diesem getrennt werden kann.

9. Ausstoßwerkzeug (1) zur Abgabe eines flüssigen Arzneimittels gemäß Anspruch 8, wobei in dem Zustand, in dem die Dichtung (7) in den Zylinder (20) eingesetzt ist und das Dichtungsandruckelement (8) montiert ist, der distale Endabschnitt des Kolbens (5) an dem Kolbenanschlagabschnitt (62) des Dichtungsandruckelements (8) anliegt, wenn der Kolben (5) in der distalen Richtung vorgeschoben wird, und der Montagezustand des Dichtungspresselements (8) auf der Dichtung (7) beibehalten bleibt und der Stößelanschlagabschnitt (62) von dem distalen Endabschnitt des Stößels (5) getrennt wird, wenn der Stößel (5) in eine proximale Richtung gezogen wird.

10. Ausstoßwerkzeug (1) zur Abgabe eines flüssigen Arzneimittels gemäß Anspruch 9, wobei, wenn der Kolben (5) in der proximalen Richtung in dem Zustand gezogen wird, in dem die Dichtung (7) in den Zylinder (20) eingesetzt ist und der Dichtungsbefestigungsabschnitt (80) in den Lumenabschnitt (70) der Dichtung (7) eingesetzt ist, die distale Oberfläche (80a) des Befestigungsabschnitts des Dichtungsbefestigungsabschnitts (80) einen Zustand des Anliegens an der distalen Oberfläche des Lumens des Lumenabschnitts (70) der Dichtung (7) aufrechterhält.

11. Ausstoßwerkzeug (1) zur Abgabe eines flüssigen Arzneimittels gemäß einem der Ansprüche 1 bis 10, wobei das Spritzenbefestigungselement (4) einen rohrförmigen Aufnahmeabschnitt (42) hat, der sich in proximaler Richtung von einem Körperabschnitt mit dem proximalen Zylinderbefestigungsabschnitt erstreckt und in der Lage ist, den Dichtungsbefestigungsabschnitt (80) aufzunehmen.

12. Ausstoßwerkzeug (1) zur Abgabe eines flüssigen Arzneimittels gemäß Anspruch 11, wobei
der Kolben (5) hat: einen Schaftabschnitt (51), der sich von dem distalen Endabschnitt in die proximale Richtung erstreckt und einen schaftseitigen Schraubabschnitt (55) an einer äußeren Umfangsfläche aufweist; und einen Griff (52), der so konfiguriert ist, dass er den Kolben (5) dreht, der an einem proximalen Endabschnitt des Schaftabschnitts bereitgestellt ist,
das Spritzenbefestigungselement (4) einen rohrförmigen Einführungsabschnitt (110) hat, der sich von dem rohrförmigen Aufnahmeabschnitt (42) in die proximale Richtung erstreckt und in den der Schaftabschnitt des Kolbens (5) eingeführt wird,
der Einführungsabschnitt einen einführungsabschnittsseitigen Verschraubungsabschnitt (46) aufweist, der mit dem schaftseitigen Verschraubungsabschnitt (55) des Schaftabschnitts an einer Innenfläche verschraubt ist, und
der Stößel (5) durch Verschraubung zwischen dem schaftseitigen Verschraubungsabschnitt (55) und dem einführungsabschnittsseitigen Verschraubungsabschnitt (46) unter Drehung des Schaftabschnitts in die distale Richtung vorgeschoben wird.

13. Werkzeug zur Verabreichung eines flüssigen Arzneimittels, mit: dem Ausstoßwerkzeug (1) zur Abgabe eines flüssigen Arzneimittels gemäß einem der Ansprüche 1 bis 12; einem in die Spritze (2) eingefüllten flüssigen Arzneimittel; und einem Dichtungselement (21), das den distalen Endabschnitt des Zylinders (20) abdichtet.

## Revendications

1. Outil d'éjection de médicament liquide (1) comprenant : une seringue (2) qui inclut un cylindre (20) ayant une bride (23) à une partie d'extrémité proximale et un joint d'étanchéité (7) pouvant coulisser à l'intérieur du cylindre (20) et fait d'un matériau élastique ; et un outil de compression de joint d'étanchéité (3) utilisé en étant monté sur la seringue (2), dans lequel
l'outil de compression de joint d'étanchéité (3) inclut : un élément de compression de joint d'étanchéité (8) ayant une partie de montage de joint d'étanchéité (80), qui est capable d'entrer dans la partie lumière (70) du joint d'étanchéité (7) et fait d'un matériau plus dur que le joint d'étanchéité (7), et une partie de compression de surface proximale de joint d'étanchéité (81) prévue à une partie d'extrémité proximale de la partie de montage de joint d'étanchéité (80) ; un élément de montage de seringue (4) qui loge de façon mobile l'élément de compression de joint d'étanchéité (8) et a une partie de montage de cylindre proximale qui est montable sur une partie d'extrémité proximale du cylindre (20) ayant la bride (23) ; et un piston (5) qui a une partie d'extrémité distale entrant dans l'élément de montage de seringue (4) et est capable de comprimer l'élément de compression de joint d'étanchéité (8) dans une direction distale, **caractérisé en ce que**
le joint d'étanchéité (7) inclut une partie tubulaire (72), une partie de fermeture distale (71) qui ferme une extrémité distale de la partie tubulaire (72), une partie lumière (70) définie par la partie tubulaire (72) et la partie de fermeture distale (71), et une nervure annulaire (73, 74, 75) prévue sur une surface extérieure de la partie tubulaire (72),
le joint d'étanchéité (7) a une longueur axiale de partie lumière d'origine S lorsque le joint d'étanchéité (7) n'est pas inséré dans le cylindre (20) et l'élément de compression de joint d'étanchéité (8) n'est pas monté, la partie de montage de joint d'étanchéité (80) de l'élément de compression de joint d'étanchéité (8) a une longueur axiale T, et la longueur axiale T est plus longue que la longueur axiale de partie lumière d'origine S, et
lorsque le joint d'étanchéité (7) est inséré dans le cylindre (20) et l'élément de compression de joint d'étanchéité (8) est monté, une surface distale de partie de montage de la partie de montage de joint d'étanchéité (80) de l'élément de compression de joint d'étanchéité (8) se met en butée sur une surface distale de lumière (76) de la partie lumière (70) du joint d'étanchéité (7), et la partie de compression de surface proximale de joint d'étanchéité (81) de l'élément de compression de joint d'étanchéité (8) se met près de, ou se met en butée sur, une surface proximale du joint d'étanchéité (7).

2. Outil d'éjection de médicament liquide (1) selon la revendication 1, dans lequel la longueur axiale T est 1,05 à 1,20 fois la longueur axiale de partie lumière d'origine S.

3. Outil d'éjection de médicament liquide (1) selon la revendication 1 ou 2, dans lequel la partie de montage de joint d'étanchéité (80) inclut : une partie d'extrémité distale ayant une surface distale (80a) de la partie de montage ; et une partie conique côté proximal (80c) qui s'étend depuis la partie d'extrémité distale de la partie de montage de joint d'étanchéité (80) jusqu'à la partie de compression de surface proximale de joint d'étanchéité (81) et est agrandie en diamètre vers un côté arrière pour avoir une forme conique, et une surface extérieure entière de la partie conique côté proximal (80c) se met près de, ou se met en butée sur, une surface intérieure de la partie lumière (70) du joint d'étanchéité (7).

4. Outil d'éjection de médicament liquide (1) selon la revendication 3, dans lequel un angle de conicité de la partie conique côté proximal de la partie de montage de joint d'étanchéité (80) est de 1 à 8 degrés.

5. Outil d'éjection de médicament liquide (1) selon l'une quelconque des revendications 1 à 4, dans lequel une surface extérieure entière de la partie de montage de joint d'étanchéité (80) a une forme qui coïncide sensiblement avec une surface intérieure entière de la partie lumière (70) du joint d'étanchéité (7) lorsqu'il est inséré dans le cylindre (20).

6. Outil d'éjection de médicament liquide (1) selon l'une quelconque des revendications 1 à 5, dans lequel
la surface distale de lumière (76) de la partie lumière (70) du joint d'étanchéité (7) a : une surface plate centrale à un centre de la surface distale de lumière (76) ; et une surface conique de partie lumière (76b) qui s'étend vers l'arrière et radialement vers l'extérieur depuis un bord extérieur de la surface plate centrale,
la partie de fermeture distale (71) du joint d'étanchéité (7) a : une surface distale de joint d'étanchéité conique (71a) dont le diamètre est réduit vers une extrémité distale ; et une partie d'épaisseur uniforme (71b) qui est prise en sandwich entre la surface distale de joint d'étanchéité (71a) et la surface conique de partie lumière et a une épaisseur sensiblement uniforme,
la surface distale de partie de montage (80a) de la partie de montage de joint d'étanchéité (80) a : une surface distale plate, qui est plate, à un centre de la surface distale de partie de montage (80a) ; et une surface extérieure de bord conique s'étendant vers l'arrière et radialement vers l'extérieur depuis un bord extérieur de la surface distale plate, et
lorsque le joint d'étanchéité (7) est inséré dans le cylindre (20) et l'élément de compression de joint d'étanchéité (8) est monté, la surface distale plate de la partie de montage de joint d'étanchéité (80) se met en butée sur la surface plate centrale de la partie lumière (70) du joint d'étanchéité (7), et la surface extérieure de bord conique de la partie de montage de joint d'étanchéité (80) se met près de, ou se met en butée sur, la surface conique de partie lumière de la partie lumière (70) du joint d'étanchéité (7).

7. Outil d'éjection de médicament liquide (1) selon l'une quelconque des revendications 1 à 6, dans lequel le joint d'étanchéité (7) a une longueur axiale d'origine Q et une longueur axiale de partie lumière d'origine S dans l'état où le joint d'étanchéité (7) n'est pas inséré dans la seringue (2) et l'élément de compression de joint d'étanchéité (8) n'est pas monté, le joint d'étanchéité (7) a une longueur axiale déformée P et une longueur axiale de partie lumière déformée R dans l'état où le joint d'étanchéité (7) est inséré dans la seringue (2) et l'élément de compression de joint d'étanchéité (8) est monté, la longueur axiale déformée P est plus longue que la longueur axiale d'origine Q, et la longueur axiale de partie lumière déformée R est plus longue que la longueur axiale de partie lumière d'origine S.

8. Outil d'éjection de médicament liquide (1) selon l'une quelconque des revendications 1 à 7, dans lequel l'élément de compression de joint d'étanchéité (8) inclut une partie butée de piston (62) qui est capable de se mettre en butée sur, et d'être séparée de, la partie d'extrémité distale du piston (5).

9. Outil d'éjection de médicament liquide (1) selon la revendication 8, dans lequel, dans l'état où le joint d'étanchéité (7) est inséré dans le cylindre (20) et l'élément de compression de joint d'étanchéité (8) est monté, la partie d'extrémité distale du piston (5) se met en butée sur la partie butée de piston (62) de l'élément de compression de joint d'étanchéité (8) lorsque le piston (5) est avancé dans la direction distale, et l'état de montage de l'élément de compression de joint d'étanchéité (8) sur le joint d'étanchéité (7) est maintenu et la partie butée de piston (62) est séparée de la partie d'extrémité distale du piston (5) lorsque le piston (5) est tiré dans une direction proximale.

10. Outil d'éjection de médicament liquide (1) selon la revendication 9, dans lequel, lorsque le piston (5) est tiré dans la direction proximale dans l'état où le joint d'étanchéité (7) est inséré dans le cylindre (20) et la partie de montage de joint d'étanchéité (80) est insérée dans la partie lumière (70) du joint d'étanchéité (7), la surface distale de partie de montage (80a) de la partie de montage de joint d'étanchéité (80) maintient un état de se mettre en butée sur la surface distale de lumière de la partie lumière (70) du joint d'étanchéité (7).

11. Outil d'éjection de médicament liquide (1) selon l'une quelconque des revendications 1 à 10, dans lequel l'élément de montage de seringue (4) inclut une partie de logement tubulaire (42) qui s'étend dans la direction proximale depuis une partie corps ayant la partie de montage de cylindre proximale et est capable de loger la partie de montage de joint d'étanchéité (80).

12. Outil d'éjection de médicament liquide (1) selon la revendication 11, dans lequel
le piston (5) inclut : une partie tige (51) qui s'étend dans la direction proximale depuis la partie d'extrémité distale et a une partie de vissage côté tige (55) sur une surface périphérique extérieure ; et une poignée (52) configurée pour mettre en rotation le piston (5), prévue à une partie d'extrémité proximale de la partie tige,
l'élément de montage de seringue (4) inclut une partie d'insertion tubulaire (110) qui s'étend dans la direction proximale depuis la partie de logement tubulaire (42) et dans laquelle la partie tige du piston (5) est insérée,
la partie d'insertion a une partie de vissage côté partie d'insertion (46), qui est vissée avec la partie de vissage côté tige (55) de la partie tige, sur une surface intérieure, et
le piston (5) est avancé dans la direction distale par vissage entre la partie de vissage côté tige (55) et la partie de vissage côté partie d'insertion (46) conjointement avec la rotation de la partie tige.

13. Outil d'administration de médicament liquide, comprenant : l'outil d'éjection de médicament liquide (1) selon l'une quelconque des revendications 1 à 12 ; un médicament liquide chargé dans la seringue (2) ; et un élément d'étanchéité (21) assurant l'étanchéité de la partie d'extrémité distale du cylindre (20).
